# EUROPEAN PATENT APPLICATION

(11) **EP 3 216 868 A1**
(43) Date of publication of application: **13.09.2017**
(21) Application number: 15857426.9
(22) Date of filing: 05.11.2015
(51) Int. Cl.: C12N 15/09, A61K 35/14, A61K 35/76, C12N 5/10, C12N 7/01

(54) **VIRUS VECTOR, CELL, AND CONSTRUCT**

(30) Priority: 05.11.2014 JP 2014225642
(71) Applicant: Kyushu University National University Corporation, Fukuoka 812-8581 (JP)
(72) Inventor: TANI, Kenzaburo, Fukuoka-shi Fukuoka 812-8581 (JP); HIRAMOTO, Takafumi, Fukuoka-shi Fukuoka 812-8581 (JP); TAKEDA, Makoto, Shinjuku-ku Tokyo 162-8640 (JP); TAHARA, Maino, Tokyo 162-8640 (JP)
(74) Representative: Epping - Hermann - Fischer
(86) International application number: PCT/JP2015/081145
(87) International publication number: WO 2016/072446

(57) **Abstract**

A virus vector contains a genome that is derived from a virus belonging to the family Paramyxoviridae, and has a gene encoding a modified H protein, a gene encoding a modified F protein, and foreign genes. The genome may be segmented into multiple segments, and each genome segment may have a leader sequence and a trailer sequence.

## Description

### Technical Field

The present disclosure relates to a virus vector, a cell, and a construct.

### Background Art

Because of low invasiveness upon collection and administration, blood cells are most frequently applied clinically as therapeutic cells for regenerative medicine or the like. Particularly hematopoietic stem cells and hematopoietic progenitor cells have been used for treatments of hematopoietic malignancies over the past about 30 years, and are currently used for general treatments. Clinical trials have been conducted using, in addition to these cells, blood cells including lymphocytes, NK cells, NKT cells, dendritic cells, and the like, as cells for immunocellular therapy against malignant cells.

Furthermore in recent years, with regard to gene therapy using gene-modified blood cells, clinical trials have been conducted concerning severe congenital diseases or malignancies. In particular, treatments using gene-modified T cells transduced with a T cell receptor or chimeric antigen receptor gene have received attention as novel treatments with potentially significant therapeutic effects against malignancy.

Currently, a lentivirus vector or a retrovirus vector is used for transduction into T cells. However, such a lentivirus vector and a retrovirus vector are problematic in genomic toxicity due to insertion into genomic DNA.

Conversely, methods known as transduction methods that unlikely cause insertion to genomic DNA include methods using adenovirus vectors, as well as methods using non-viral vectors such as mRNA and plasmids. However, adenovirus vectors and non-viral vectors are problematic in extremely low transduction efficiency and limited cell types that can be used.

The use of a Sendai virus vector is also known as a transduction method that unlikely causes insertion into genomic DNA. For example, Patent Literature 1 discloses a Sendai virus vector modified through deletion of an F gene, so as to avoid the release of secondary infectious viral particles. Furthermore, Patent Literature 2 discloses a Sendai virus vector carrying a gene involved in cell reprogramming.

Measles virus belonging to the family Paramyxoviridae, to which Sendai virus also belongs, is a virus retaining extremely high infectivity to immunocytes and epithelial cells. Patent Literature 3 discloses measles virus retaining multiple genome segments, at least one of which contains a foreign gene that can be expressed within a host.

### Citation List

### Patent Literature

Patent Literature 1: Japanese Patent No. 3602058
Patent Literature 2: International Publication No. WO 2010/008054
Patent Literature 3: International Publication No. WO 2007/007921

### Summary of Invention

### Technical Problem

When a gene is introduced into a T cell, the Sendai virus vectors disclosed in Patent Literature 1 and Patent Literature 2 above are inconvenient in that the construction of the Sendai virus vectors is relatively difficult, and Sendai virus tends to remain and the removal thereof is difficult. Moreover, there is less knowledge about how the Sendai virus vectors affect humans that are not original hosts for Sendai virus. Actual clinical use of the Sendai virus vectors should be carefully performed because of safety concerns.

Furthermore, similarly to adenovirus vectors, in the case of the Sendai virus vectors disclosed in the above Patent Literature 1 and Patent Literature 2 and the measles virus disclosed in the above Patent Literature 3, T cells should be stimulated and activated prior to transduction in order to increase transduction efficiency. This causes a concern of inducing T cell differentiation due to T cell activation, and thus transduction into naive T cells, undifferentiated B cells, or the like is difficult.

As described above, conventional virus vectors are problematic in that the safe use of these vectors for humans is insufficiently ensured and cells into which genes can be efficiently introduced by the vectors are limited.

The present disclosure has been achieved in view of the above circumstances and an objective thereof is to provide a virus vector, a cell, and a construct that are highly safe and enables efficient introduction of genes into wide-ranging cells.

### Solution to Problem

A virus vector according to a first aspect of the present disclosure includes
a genome derived from a virus belonging to the family Paramyxoviridae in which both a gene encoding an H protein and a gene encoding an F protein are modified, wherein
the genome contains a foreign gene.

In this case, the genome
may be segmented into multiple segments, wherein
each genome segment may include a leader sequence and a trailer sequence.

Furthermore, the genome may be segmented into two segments.

The modifications may be
a deficiency in the gene encoding the H protein, or a mutation resulting from substitution, deletion, or addition of one or several amino acids of the H protein, and
a deficiency in the gene encoding the F protein, or a mutation resulting from substitution, deletion, or addition of one or several amino acids of the F protein.

Furthermore, a gene encoding an M protein in the genome may include,
a mutation resulting from substitution, deletion, or addition of one or several amino acids of the M protein.

Furthermore, the virus belonging to the family Paramyxoviridae may be measles virus.

Furthermore, the foreign gene may include
OCT3/4, SOX2 and KLF4.

A cell according to a second aspect of the present disclosure is a cell, wherein
the foreign gene is introduced by the virus vector according to the first aspect of the present disclosure.

In this case, the cell according to the second aspect of the present disclosure may be a blood cell including a hematopoietic stem cell.

In addition, the blood cell may be
a naive T cell, a stem cell-like memory T cell, or an undifferentiated B cell.

Furthermore the foreign gene may include,
OCT3/4, SOX2 and KLF4, and
the cell may be a pluripotent stem cell in a ground state.

A construct according to a third aspect of the present disclosure, contains
a nucleic acid that is a template for multiple genome segments derived from a virus belonging to the family Paramyxoviridae, in which a leader sequence is located at the 3' end and a trailer sequence is located at the 5' end, respectively, wherein
both the gene encoding the H protein and the gene encoding the F protein in the segmented genome are modified.

### Advantageous Effects of Invention

According to the present disclosure, a gene can be efficiently introduced into wide-ranging cells with a high level of safety.

### Brief Description of Drawings

FIG. 1 shows the composition of a segmented genome of a non-transmissible measles virus vector carrying six genes;
FIG. 2 shows the composition of the measles virus vector plasmid shown in FIG. 1;
FIG. 3 shows fluorescent images of BJ cells and activated T cells into which measles virus vectors were introduced;
FIG. 4 shows the results of analyzing the expression of genes carried by the measles virus vector;
FIG. 5 shows the transduction efficiencies of the measles virus vector and a Sendai virus vector into cells of each blood cell lineage;
FIG. 6 shows the transduction efficiencies of the measles virus vector and the Sendai virus vector into cord blood-derived T cells;
FIG. 7 shows the transduction efficiencies of the measles virus vector and the Sendai virus vector into each fraction of peripheral blood-derived T cells;
FIG. 8 shows the phase difference and fluorescent images of the colonies of induced pluripotent stem cells (iPS cells) established from BJ cells;
FIG. 9 shows the phase difference and fluorescent images of the primary colonies of iPS cells established from T cells;
FIG. 10 shows the expression of undifferentiation markers in BJ cell-derived iPS cells;
FIG. 11 shows the results of analyzing the expression of undifferentiation markers and virus-derived genes by an RT-PCR method;
FIG. 12 shows the results of analyzing the *in vitro* induction of triploblastic differentiation;
FIG. 13 shows the results of analyzing triploblastic differentiation ability by a teratoma formation test;
FIG. 14 shows the results of analyzing the karyotype of BJ cell-derived iPS cells; and
FIG. 15 shows the morphology of iPS cells in a ground state.

### Description of Embodiments

Embodiments according to the present disclosure will be explained as follows.

### (Embodiment 1)

First, Embodiment 1 is explained in detail. A virus vector according to this embodiment contains a genome derived from a virus belonging to the family Paramyxoviridae.

The virus belonging to the family Paramyxoviridae (hereinafter, also simply referred to as "virus") is an RNA virus having a (-) strand RNA genome. Examples of the virus include Measles virus, Sendai virus, Newcastle disease virus, Mumps virus, Parainfluenza virus 1, Parainfluenza virus 2, Parainfluenza virus 3, Parainfluenza virus 5 or Simian virus 5, Metapneumo virus, Respiratorysyncytial virus, Rinderpest virus, and Distemper virus. The virus belonging to the family Paramyxoviridae is preferably measles virus.

The genome of the virus belonging to the family Paramyxoviridae is unsegmented single-stranded RNA, having a leader sequence (Le) at the 3' end and a trailer sequence (Tr) at the 5' end. Various genes encoding functional proteins that constitute the virus are located between the leader sequence and the trailer sequence. The leader sequence has promoter activity. The trailer sequence has promoter activity in the case of antigenome.

Major genes encoding the functional proteins are a gene encoding an N protein (N gene), a gene encoding a P protein (P gene), a gene encoding an M protein (M gene), a gene encoding an F protein (F gene), a gene encoding an H protein (H gene), and a gene encoding an L protein (L gene). Transcriptional regulatory sequences; that is, a transcription initiation sequence and a transcription termination sequence, are present on both ends of each of the six genes in the genome.

The N proteins are systematically aligned and bound in order from the 5' end with respect to the viral genome, so as to package the genome RNA. The P protein functions as a small subunit of RNA-dependent RNA polymerase, and is involved in transcriptional replication of the viral genome. In addition, accessory proteins such as a V protein and a C protein may be synthesized from the P gene through RNA editing mechanism or the like. The L protein functions as a large subunit of RNA-dependent RNA polymerase, and is involved with the P protein in transcriptional replication of the viral genome.

The M protein is a matrix protein supporting from inside the structure of viral particles. The F protein is involved in cell fusion, and imparts pathogenicity to a virus. The H protein is a viral receptor-binding protein. The H protein binds to signaling lymphocyte activation module (SLAM) or Nectin4 as a receptor for a wild-type virus to infect. SLAM and Nectin4 are expressed only in limited cell types, thereby limiting viral host range.

The term "genome derived from a virus belonging to the family Paramyxoviridae" means a genome that has genes encoding the functional proteins, and can be amplified by infected cells.

In the above genome contained in the virus vector according to this embodiment, both the H gene and the F protein are modified. Modification of the H gene is a deficiency in the H gene, for example. Deletion of the H gene can cause the absence of the expression of the H protein. Moreover, modification of the H gene may be a mutation resulting from substitution, deletion, or addition of one or several amino acids of the H protein.

More specifically, examples of the modification of the H gene include, substitution of the 390^{th} asparagine with isoleucine (N390I), substitution of the 416^{th} asparagine with aspartic acid (N416D), substitution of the 446^{th} threonine with serine (T446S), substitution of the 481^{st} asparagine with tyrosine (N481Y) and substitution of the 492^{nd} glutamic acid with glycine (E492G) of the H protein. Because of these substitutions, the H protein can bind to a membrane co-factor protein (CD46), the expression of which is observed in almost all cells, in addition to SLAM and Nectin4.

The structure or the functions of the H protein can be changed with respect to the wild-type H protein through the modification of the H gene. Since the H protein is a viral receptor-binding protein as described above, the virus vector can use molecules other than SLAM and Nectin4 as receptors through modification of the H gene. Specifically, modification of the H gene can result in increased number of cell types that the virus vector can infect.

Modification of the F gene is deficiency in the F gene, for example. Deletion of the F gene can cause the absence of the expression of the F protein. For example, when the genome of measles virus is used, the F protein-deficient virus vector is unable to produce any pathogenic virus within cells other than special cell lines (Vero/SLAM/F cell). Hence, unless caused to infect the above special cell lines, the virus vector can be caused to lose the transmission.

Modification of the F gene may be a mutation resulting from substitution, deletion, or addition of one or several amino acids of the F protein. The structure or the functions of the F protein may be changed with respect to the wild-type F protein through the modification.

Furthermore, the gene encoding the M protein in the above genome may have a mutation resulting from substitution, deletion, or addition of one or several amino acids of the M protein. Examples of the mutation include substitution of the 64^{th} proline with serine (P64S) and substitution of the 89^{th} glutamic acid with glycine (E89G) of the M protein. Since the M protein is a matrix protein supporting from inside the structure of viral particles, the ability to form viral particles can be improved and the cell fusion ability can be lowered by mutating the M protein.

The above genome contained in the virus vector according to this embodiment has foreign genes. Therefore, the virus vector enables the expression of the foreign genes. Examples of the foreign genes include, but are not limited to, genes encoding various proteins of viruses, bacteria, parasites and the like, which induce pathogenicity, genes encoding various cytokines, genes encoding various peptide hormones, and genes encoding cell reprogramming factors. In addition, as a foreign gene, a reporter gene such as a gene encoding a Green Fluorescent Protein (GFP) or an Enhanced Green Fluorescent Protein (EGFP) may be inserted.

The number of foreign genes is not particularly limited, and preferably ranges from 2 to 6. Specific examples of foreign genes include reprogramming factors such as OCT3/4, SOX2 and KLF4. Other reprogramming factors such as L-MYC and PIN1 may of course be further inserted as foreign genes into a genome.

The positions of genes encoding the above functional proteins and foreign genes in a genome are not particularly limited, as long as the positions are between a leader sequence and a trailer sequence. The mutual positional relationship among genes encoding the above functional proteins in a genome can be arbitrarily determined regardless of positions such as the order of alignment in a wild-type viral genome.

The above genome contained in the virus vector according to this embodiment may be segmented into multiple segments. The term "segmentation" means that genome RNA is fragmented into multiple RNA fragments. Specifically, multiple genome segments form a set of RNA groups that function as one genome. The number of genome segments is not limited, but is preferably up to 6, and is particularly preferably 2.

In this case, each genome segment has a leader sequence and a trailer sequence. Preferably, a leader sequence is located at one end and a trailer sequence is located at the other end of each genome segment. In this case, any one of genes encoding the above functional proteins, and foreign genes are placed between the leader sequence and the trailer sequence of each genome segment.

Genes encoding functional proteins and foreign genes may be inserted into one, two or more of multiple genome segments. When multiple foreign genes are expressed, all the multiple foreign genes may be inserted into one of multiple genome segments. Alternatively, one or more foreign genes may be inserted into each of two or more of multiple genome segments, so as to enable the insertion of multiple foreign genes overall. When multiple foreign genes are inserted into one of multiple genome segments, the number of foreign genes is not limited as long as the number is within the range that does not cause any significant decrease in the expression efficiency of genes encoding the above functional proteins.

The positions of foreign genes in multiple genome segments are not particularly limited. For example, when the genome of measles virus is segmented into two segments to cause deficiency in the F gene, and reprogramming factors are inserted as foreign genes, the resulting first genome segment contains a leader sequence, OCT3/4, the N gene, the P gene, a modified M gene, SOX2, and a trailer sequence that are placed in this order from the 3' end to the 5' end, and the resulting second genome segment contains a leader sequence, KLF4, a modified H gene, the L gene, and a trailer sequence are placed in this order from the 3' end to the 5' end. When L-MYC and PIN1 are further inserted as foreign genes, preferably, L-MYC may be inserted between SOX2 and a trailer sequence, and PIN1 may be inserted between the H gene and the L gene.

Genes encoding the above functional proteins, excluding the H gene, the F gene, and the M gene, may be mutated genes, each comprising a nucleotide sequence that is not completely the same as that of the corresponding wild-type gene contained in the genome of the wild-type virus, as long as the activity upon transcription and replication is equivalent to or higher than the wild-type activity. The nucleotide sequences of genes encoding functional proteins may be the nucleotide sequences encoding proteins, each comprising amino acids resulting from deletion, substitution, or addition of one or several (such as 1 to 15, preferably 1 to 8, and more preferably 1 to 5) amino acids, as long as the activity upon transcription and replication is equivalent to or higher than that of each natural protein. The mutant proteins and the natural proteins have amino acid sequence homology of preferably 90% to 100%, for example, and the amino acid homology may range from 40% to 90%, for example, as long as the activity upon transcription and replication is retained.

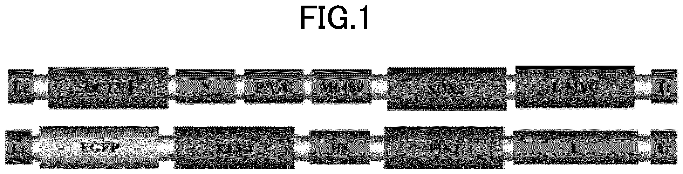

FIG. 1 shows a preferred example of the above segmented genome having genes encoding reprogramming factors and a gene encoding EGFP as foreign genes. In the genome segmented into two segments, the H gene is modified (H8) so as to add N390I, N416D, T446S, N481Y and E492G mutations to the H protein, and the F gene is deficient. Furthermore, the M gene is modified (M6489) so as to add P64S and E89G mutations to the M protein. In addition, the P gene is represented by P/V/C.

The virus vector has ability to infect cells and transmissibility. The expression "ability to infect cells" refers to ability to retain adherence of the virus vector to host cells and membrane fusion ability, so as to be able to introduce the viral genome or the like into cells. Furthermore, the term "transmissibility" refers to ability to replicate the genome introduced into cells, and to form infectious particles or complexes similar thereto, so as to be able to transmit the genome to other cells.

The virus vector according to this embodiment can be constructed by a known method for constructing a virus vector. For example, the virus vector can be constructed by rearranging viral particles using cDNA corresponding to a viral genome, or using a viral genome. Here, the expression "rearranging viral particles" means to artificially prepare a viral genome, so as to prepare the original virus or a recombinant virus *in vitro* or within cells.

Any of these methods generally involves isolating the genome from a virus, and then preparing cDNA of the genome via reverse transcription reaction or the like. When the genome is subsequently segmented, the cDNA is fragmented into multiple cDNA fragments by a known genetic recombination technique, a nucleic acid amplification method and the like, and then the resultant is subjected to a procedure such as insertion of a foreign gene. A method for fragmentation is not limited, as long as multiple cDNA fragments are thus prepared by the method based on the nucleotide sequence or the like of the cDNA prepared from the genome.

An example of a preparation method based on the nucleotide sequence of cDNA prepared from a genome is a method that involves obtaining an amplification fragment by a PCR method or the like using as a template a predetermined region in cDNA prepared from a genome. The predetermined region can be set as appropriate in view of the structure of a genome to be expressed, and is not limited. The predetermined region is preferably set, so that gene fragments encoding viral functional proteins can be separately amplified. When the region is set as described above, the desired types and number of the thus obtained amplified fragments of each gene are bound in order (at positions) as desired, so that cDNA fragments can be prepared.

A foreign gene may be inserted into a segmented genome by inserting a DNA fragment containing a separately prepared foreign gene into the above segmented cDNA using a known genetic recombination technique. A cDNA nucleotide sequence corresponding to the first genome segment containing the N gene and a cDNA nucleotide sequence corresponding to the second genome segment containing the H gene in FIG. 1 are shown in SEQ ID NO: 1 and SEQ ID NO: 2, respectively.

Viral particles may be rearranged using DNA containing a prepared cDNA fragment, preferably plasmid DNA, or, RNA obtained by prior *in vitro* transcription of cDNA, for example.

In general, direct introduction of the genome of a virus belonging to the family Paramyxoviridae or the antigenome thereof in the form of naked RNA into host cells does not lead to obtainment of a template for RNA-dependent RNA polymerase. Obtainment of a template requires the presence of the N protein, the P protein and the L protein at the initial stage of an RNA synthetic reaction conducted by the RNA-dependent RNA polymerase, and the formation of a complex (RNP complex) of these proteins and genome RNA. Accordingly, for rearrangement of a virus vector, the N protein, the P protein and the L protein are desirably expressed together, or, a host capable of expressing the N protein, the P protein and the L protein is desirably used. In addition to this, when the F gene is deleted in RNA contained in the above virus vector, a host capable of expressing also the F protein is preferably used.

The above virus vector can be constructed by, for example, introducing the above genome or the cRNA of the genome into a host expressing viral N protein, P protein and L protein. Furthermore, DNA containing the above genome or cDNA that serves as a template for the cRNA of the genome and a transcription unit of the DNA may be introduced into a host expressing viral N protein, P protein and L protein. Moreover, DNA containing the above genome or cDNA that serves as a template for the cRNA of the genome, DNA containing viral N gene, DNA containing viral P gene, DNA containing viral L gene, and transcription units of these DNAs may be introduced into a host.

Proteins to be used as the above N protein, P protein and L protein that are expressed by a host may be proteins that are not completely the same as the natural proteins, as long as the proteins have activity equivalent to or higher than that of the natural proteins. For example, the N, P and L proteins may be proteins comprising amino acids resulting from deletion, substitution or addition of 1 or several (such as 1 to 15, preferably 1 to 8, and more preferably 1 to 5) amino acids, or, completely different proteins derived from other viruses or comprising significantly different amino acid sequences.

The above host is not particularly limited, as long as it is a cell capable of expressing the above functional proteins and foreign genes. Examples thereof include cultured mammalian or avian cells, and a chicken egg. Specific examples of cultured cells that can be used herein include BHK-T7/9 cells, CHO, 293 cells, B95a, monkey cells such as COS-7 and Vero, mouse L cells, rat GH3, human FL cells, LLCMK2, MDCK, MDBK, CV-1, HeLa, HepG2, P19, F9, PC1Z, BAF3, Jurkat, human PBMCN, MT-4, Molt-4, NIH3T3, L929, Vero/hSLAM, CHO/hSLAM, A549/hSLAM, HeLa/hSLAM, 293T, BHK, and chicken embryonic fibroblasts. Furthermore, insect cells such as Sf9 cells, and Sf21 cells can also be used.

As the above transcription unit, for example, recombinant vaccinia virus expressing predetermined DNA-dependent RNA polymerase, DNA containing a predetermined DNA-dependent RNA polymerase gene, and the like are preferred.

Furthermore, a virus vector that is obtained by the above manufacturing methods can be replicated selectively and efficiently by co-culture with other cells. For example, in the case of a virus vector constructed using measles virus, cultured cells containing a rearranged virus vector obtained by the above manufacturing methods are seeded on Vero/SLAM/F cells cultured in advance, and then co-cultured, so that giant cells of Vero/SLAM/F cells can be obtained as a result of infection and proliferation of the virus vector.

More specifically, for example, when a measles virus vector is constructed, two plasmids corresponding to genome segments shown in FIG. 1 may be introduced together with another plasmid containing genes encoding functional proteins if necessary, into appropriate cells. Subsequently, cells are recovered and seeded on Vero/SLAM/F cells, and then the thus expressed giant cells are collected, so that a measles virus vector can be obtained. The measles virus vector is caused to infect fresh Vero/SLAM/F cells for amplification, and then the measles virus vector may be released. In such a case, a supernatant containing the relevant virus can be recovered as a viral solution by centrifugation.

As described in detail above, the virus vector according to this embodiment contains a modified H gene, so that the virus vector can infect various cell types, and specifically can broaden the host range. Furthermore, the virus vector contains a modified F gene, so as to be able to prevent the production of the virus having ability to infect cells, and to cause the virus to lose the transmission of the virus vector and thus to improve safety. Moreover, the above virus vector carries out all the replication processes within cytoplasm without DNA synthesis, so that the virus vector has no concern of genomic toxicity, and thus has an extremely high level of safety.

In addition, a genome to be contained in the virus vector according to this embodiment may be segmented into multiple segments. In this manner, the virus vector can carry multiple foreign genes or a gene having a large size. Moreover, each of the genome segments may have a leader sequence and a trailer sequence. Viruses belonging to the family Paramyxoviridae are known to have a characteristic gene expression pattern such that the more downstream (5' side) location of a gene, the lower the expression of the gene. Each of genome segments has a leader sequence and a trailer sequence, so that polymerase can act on each genome segment, and the expression level of each gene can be increased. Furthermore, multiple foreign genes can be expressed simultaneously in a host. Moreover, segmentation of a genome into two segments leads to a genome size appropriate for maintaining the protein expression efficiency at a high level. An unsegmented single genome can of course exert similar functions.

In addition, in this embodiment, a gene encoding the M protein on a segmented genome may comprise a mutation resulting from substitution, deletion, or addition of one or several amino acids of the M protein. In this manner, the transduction efficiency and the safety of the virus vector for cells can be even more enhanced.

Furthermore, in this embodiment, a virus may be measles virus. Measles virus has strong tropism to immunocytes. Hence, the above virus vector enables highly efficient transduction into B cells, T cells and granulocytes. Furthermore, the above virus vector enables, as described in Examples below, extremely highly efficient transduction into inactivated naive T cells, central memory T cells, effector memory T cells and B cells, and even blood cells including hematopoietic stem cells. Moreover, the original host of the measles virus is a human, and knowledges concerning the effects of the measles virus on humans have been accumulated, and thus the measles virus can be appropriately used with due attention on safety aspects.

In addition, in this embodiment, examples of foreign genes include OCT3/4, SOX2 and KLF4. When the genome of measles virus is used as a genome of the virus vector according to this embodiment, iPS cells in a ground state, the preparation of which has been extremely difficult by a conventional method, can be prepared using the virus vector carrying genes encoding reprogramming factors.

In addition, RNA to be contained in the virus vector according to this embodiment may be, in addition to (-) strand RNA, (+) strand RNA as necessary, similarly to the original genome of the virus.

In another embodiment, a construct appropriate for preparation of a genome to be contained in the above virus vector is provided. The construct contains nucleic acids serving as templates for multiple segments of a genome derived from a virus belonging to the family Paramyxoviridae, in which a leader sequence is located at the 3' end and a trailer sequence is located at the 5' end, respectively, wherein both the gene encoding the H protein and the gene encoding the F protein are modified in the segmented genome.

The above nucleic acids may be cDNAs or cRNAs, which are prepared based on each of the above genome segments. For example, the nucleic acids may be multiple cDNAs or plasmid DNAs corresponding to each of the genome segments.

For example, when the genome of measles virus is segmented into two segments to cause deficiency in the F gene, the construct contains cDNA corresponding to a genome in which a leader sequence, the N gene, the P gene, the M gene and a trailer sequence are located in order from the 3' end to the 5' end, and cDNA corresponding to a genome in which a leader sequence, the H gene, the L gene and a trailer sequence are located in order from the 3' end to the 5' end.

This construct facilitates insertion of foreign genes using a genetic recombination technique of conventional genetic engineering. With the use of the construct for rearrangement of viral particles as described above, the above virus vector containing each genome segment can be efficiently constructed.

This construct can be introduced into prokaryotic cells or eukaryotic cells by a conventional transformation or transfection technique. For example, this expression construct can be introduced into various types of cells, in the form of plasmids.

In addition, nucleic acids to be contained in the above construct may be cRNAs of multiple segments of a genome derived from a virus belonging to the family Paramyxoviridae, in which a leader sequence is located at the 3' end and a trailer sequence is located at the 5' end, respectively. Moreover, the above construct contains nucleic acids serving as templates for the genome derived from a virus belonging to the family Paramyxoviridae, in which a leader sequence is located at the 3' end and a trailer sequence is located at the 5' end, wherein both the gene encoding the H protein and the gene encoding the F protein in the genome may be modified.

### (Embodiment 2)

Next, Embodiment 2 is explained. Cells according to this embodiment are transduced with foreign genes by the virus vector according to the above Embodiment 1.

The virus vector can be caused to infect cells by a known method. For example, the virus vector is added to a culture solution containing cells, and then the solution may be centrifuged at room temperature at 1,200 x g for 45 minutes. Further examples of a method for causing the virus vector to infect cells include electroporation, lipofection, a heat shock method, a PEG method, a calcium phosphate method and a DEAE dextran method, which involve causing various types of virus to infect cells.

Examples of cells are not particularly limited and include human somatic cells, fibroblasts, and blood cells, as well as monkey somatic cells. Preferred examples of cells include B cells, activated or inactivated T cells, granulocytes, and blood cells including hematopoietic stem cells. Examples of particularly preferred cells include naive T cells, stem cell-like memory T cells or undifferentiated B cells. The titer of the virus vector upon transduction of cells with foreign genes is not particularly limited, and multiplicity of infection (MOI) ranges from 1 to 100, preferably 3 to 20, and more preferably 5 to 10.

Cells transduced with foreign genes by the above virus vector containing at least OCT3/4, SOX2 and KLF4 as the foreign genes express an undifferentiation marker, and are induced to be iPS cells having triploblastic differentiation ability. iPS cells in a ground state can also be induced through transduction by the above virus vector.

As explained in detail above, cells according to the embodiment can express desired multiple foreign genes simultaneously for a long time period, since the genes are introduced by the above virus vector. Moreover, naive T cells, stem cell-like memory T cells or undifferentiated B cells, and even hematopoietic stem cells can be selected as subjects of transduction, so that cells according to this embodiment is extremely useful for gene therapy using gene-modified blood cells, and particularly gene-modified T cell therapy.

Moreover, the cells are transduced with foreign genes by the above virus vector containing OCT3/4, SOX2 and KLF4 and then induced to be iPS cells in a ground state. Such iPS cells in a ground state have even higher proliferation efficiency and are capable of proliferating even when seeded in their unicellular form, so that the iPS cells can be prepared in large amounts and easily stored. Moreover, because of the ground state, the iPS cells can be differentiated into wide-ranging cell types.

In addition, applications of the cells according to this embodiment are varied depending on foreign genes. The cells can be used for T cell therapy against malignancy through the use of a T cell receptor gene or a chimeric antigen receptor gene as a foreign gene to be introduced into the cells. Furthermore, Zinc finger nuclease or the like is introduced as a foreign gene into T cells so as to disrupt (gene editing) a CCR5 gene, and thus the cells can be used for treatment of HIV/AIDS. Enzyme deficiency can also be treated by introducing a gene encoding an enzyme; that is, adenosine deaminase (ADA) or the like into cells of a patient with enzyme deficiency in adenosine deaminase (ADA) or the like.

In addition, in another embodiment, a method for preparing iPS cells is provided. The method for preparing iPS cells comprises an infection step for causing the virus vector according to the above Embodiment 2 to infect cells. The cells may be blood cells, inactivated (unstimulated) immunocytes, and are preferably naive T cells. Examples of the method for preparing iPS cells include methods for preparing iPS cells in a ground state.

### Examples

The present disclosure will be further specifically described with reference to the following examples, but the present disclosure is not limited by the examples.

### (Cell culture)

Cell culture in the following examples was performed as follows. Mouse embryonic fibroblasts (MEF cells), Vero/SLAM cells, and BJ cells of a fibroblast cell line were cultured in a culture solution of Dulbecco's modified eagle's medium (DMEM) (NACALAITESQUE, INC.) containing 10% fetal bovine serum (FBS) (HyClone). Vero/SLAM/F cells were cultured in a culture solution of DMEM containing 0.5 mg/mL G418 (NACALAI TESQUE, INC.) and 7.5% FBS.

BHK-T7/9 cells were cultured in a culture solution of α-modified minimum essential medium eagle (α-MEM) (Life Technologies Corporation) containing 600 µg/mL Hygromycin B (NACALAI TESQUE, INC.) and 10% FBS. Peripheral blood-derived blood cells of a healthy individual were cultured in a culture solution of RPMI1640 (NACALAITESQUE, INC.) containing 10% FBS. Cord blood-derived or healthy individual-derived T cells were cultured in a culture solution of RPMI1640 containing 10% FBS, the same culture solution supplemented with 175 IU/mL human recombinant IL-2 (PeproTech, Inc.), or a KBM502 culture solution (Kohjin Bio Co., Ltd.). Cord blood-derived hematopoietic stem cells were cultured in StemSpan culture solution (Veritas Corporation) supplemented with human recombinant SCF, human recombinant Flt-3L and human recombinant TPO (all produced by PeproTech, Inc.).

All primed-type iPS cells were cultured on MEF cells in a culture solution for maintaining human ES cells. The composition of the culture solution for maintaining human ES cells is DMEM/F12 (NACALAITESQUE, INC.) containing 20% KSR (Life Technologies), 2-mercaptoethanol (Sigma), 2 mM L-glutamine (NACALAI TESQUE, INC.), 1% non-essential amino acids (NACALAI TESQUE, INC.), and 4 ng/mL basic FGF (PeproTech, Inc.).

iPS cells in a ground state were cultured on MEF cells in a mixed culture solution. The composition of the mixed culture solution is DMEM /F12 containing 20% KSR, 2-mercaptoethanol, 2mM L-glutamine, 1% non-essential amino acid, 1µM CHIR99021 (Militenyi Biotec K.K.), 1µM PD0325901 (Militenyi Biotec K.K.), and 1,000 units/mL human recombinant LIF (NACALAI TESQUE, INC.).

### (Example 1: Construction of non-transmissible gene-modified measles virus vector)

Non-transmissible gene-modified measles virus vector was produced by co-culture of two plasmids (MV-HL-K-Pin1-EGFP and MV-NPM-OSM) containing genes encoding measles-virus-constituting functional proteins and four plasmids (pCITE-IC-N, pCITE-IC-PΔC, pCITEko-9301B-L, pCA7-IC-F) that help viral synthesis, with Vero/SLAM/F cells prepared as package cells by introducing a SLAM gene and the F gene of measles virus into BHK-T7/9 cells, as described below. As foreign genes, an OCT3/4 gene, an SOX2 gene, an L-MYC gene, a KLF4 gene and a PIN1 gene to be used for iPS cell preparation, and an EGFP gene as a reporter gene were inserted.

First, for insertion of N390I, N416D, N481Y, and E492G mutations into the H protein, and for insertion of P64S and E89K mutations into the M protein, wild-type H and M genes of a measles virus strain, MV-IC323-EGFP, were modified by genetic recombination.

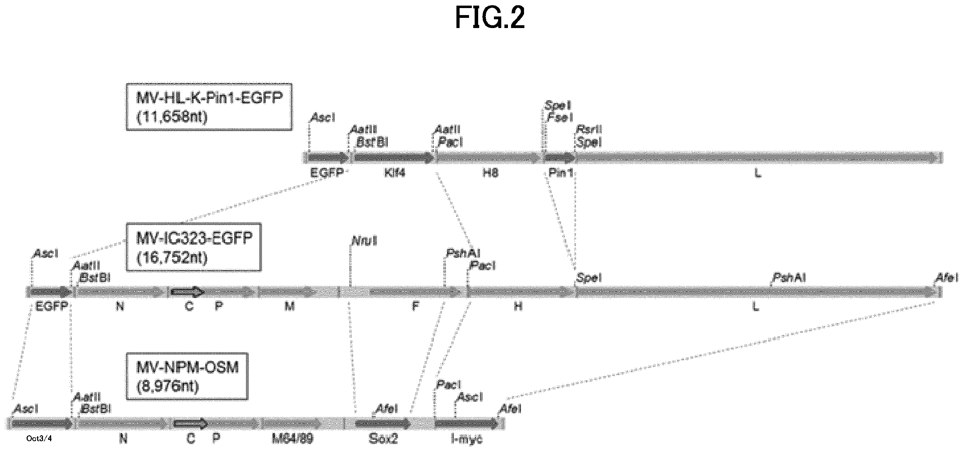

Subsequently, as shown in Fig. 2, the MV-NPM-OSM plasmid was prepared by recombining the EGFP gene of the above-mutated MV-IC323-EGFP with the OCT3/4 gene, the F gene of the same with the SOX2 gene, the H gene and the L gene of the same with the L-MYC gene. Meanwhile, the MV-HL-K-Pin1-EGFP plasmid was prepared by recombining the N gene, the P gene, the M gene and the F gene of the above mutated MV-IC323-EGFP with the KLF4 gene, and then inserting the PIN1 gene between the L gene and the H gene.

The MV-NPM-OSM plasmid and the MV-HL-K-Pin1-EGFP plasmid, and four plasmids (pCAG-T7-IC-F, pCITE-IC-N, pCITE-IC-PΔC, pCITE-ko-9301B-L) were used for transduction into BHK-T7/9 cells. Two days later, cells were recovered from dishes, and then seeded on Vero/SLAM/F cells. Two days later, giant cells were collected and a measles virus vector was recovered. The recovered measles virus vector was caused to infect fresh Vero/SLAM/F cells, and then after amplification, cells were recovered. Recovered cells were re-suspended in DMEM, followed by repeated freezing and thawing. Thus, the measles virus vector (MVV) was released from within cells into the culture solution. Next, a supernatant alone was recovered as a viral solution by centrifugation, dispensed, and then stored at -80°C. Titer was determined by adding the viral solution to Vero/SLAM cells, and then 2 days later, analyzing the proportion of GFP positive cells by a flow cytometric method.

### (Example 2: Evaluation of non-transmissible gene-modified measles virus vector)

MW prepared in Example 1 was evaluated using activated T cells or BJ cells. Activated T cells were prepared by stimulating healthy individual-derived peripheral blood with Dynabeads (Registered Trademark) Human T-Activator CD3/CD28 (Life Technologies Corporation) in a KBM502 culture solution for 5 days. More specifically, 5×10⁴ cells of each cell type were seeded in a 12-well plate, and then a viral solution in an amount corresponding to the titer was added. Next, after transduction by a centrifugation method (room temperature, 1,200×g, 45 minutes), cells were washed once with PBS, and then culture solutions were exchanged. Two days later, GFP positive cells of activated T cells were analyzed for the expression of five genes (OCT3/4, SOX2, KLF4, L-MYC and PIN1) by a flow cytometric method. Moreover, BD Cytofix/Cytoperm (Trademark) Fixation/Permeabilization Kit (BD) was used to analyze the expression of proteins derived from foreign genes. Antibodies used herein were an anti-OCT3/4 antibody, an anti-SOX2 antibody, an anti-KLF4 antibody (all of these antibodies were produced by Santa cruz), and an anti-MYC antibody and an anti-PIN1 antibody (all of these antibodies were produced by R&D).

### (Results)

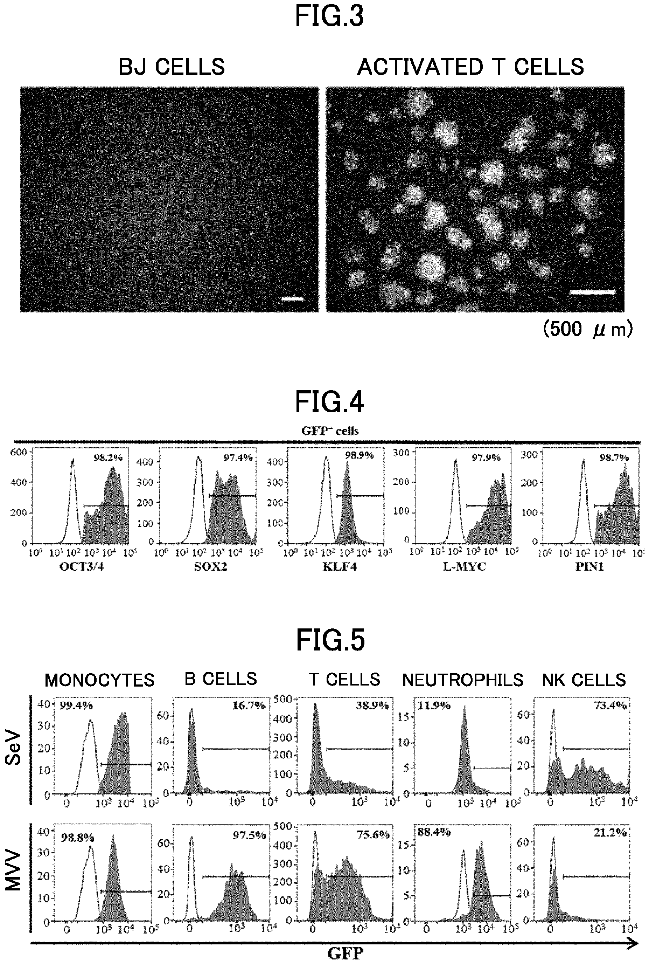

FIG. 3 shows fluorescent images of BJ cells and activated T cells. Fluorescence was confirmed for both cells. Hence, it could be confirmed that MVV conducts transduction into BJ cells and activated T cells.

FIG. 4 shows the results of analyzing GFP expression inactivated T cells. Transduction into activated T cells was performed using MW. On day 3 after transduction, the expression of the above five genes in GFP-positive cells was analyzed. The simultaneous expression of all the five genes in addition to an EGFP gene could be confirmed from the results.

### (Example 3: Transduction by non-transmissible gene-modified measles virus vector and analysis thereof)

About 10 mL of peripheral blood or cord blood was collected from a healthy individual who had consented, and then mononuclear cells were separated using a lymphocyte separation solution (NACALAI TESQUE, INC.). At the same time, about 1 ml of peripheral blood or cord blood was lysed using a hemolytic agent prepared using NH₄Cl, KHCO₃ and EDTA (all produced by NACALAI TESQUE, INC.). Blood cells recovered by these methods were subjected to transduction (MOI=5) by centrifugation (room temperature, 1,200xg, 45 minutes) using MW. After transduction, the resultant was washed with PBS(-) (prepared by dissolving PBS tablet (Takara Bio Inc.) with pure water, followed by autoclave sterilization), and then culture solutions were exchanged. Two days later, cells were recovered, and then the transduction rate of each blood cell lineage and the expression of the measles virus receptor in each cell lineage were analyzed by a flow cytometric method using GFP expression as an indicator.

As a control group for comparison, transduction was performed using a GFP gene-carrying Sendai virus vector (SeV) (PlasmEx (Registered Trademark)-AG; obtained from Medical and Biological Laboratories Co., Ltd.) by a method similar to that of MVV. Antibodies used for analysis of each blood cell lineage were monocyte lineage: an APC-Cy7-labeled anti-CD14 antibody (BioLegend, Inc.), a PE-labeled anti-CD11b antibody (BD), and a neutrophil-lineage: PerCP-Cy5.5-labeled anti-Cd15 antibody (BioLegend, Inc.), B cell lineage: an APC-Cy7-labeled anti-CD19 antibody (BioLegend, Inc.), T cell lineage: an APC-labeled anti-CD3 antibody (BioLegend, Inc.), an APC-Cy7-labeled anti-CD4 antibody (BioLegend, Inc.), a PE-Cy7-labeled anti-CD8 antibody (BioLegend, Inc.), an APC-labeled anti-CD45RA (BioLegend, Inc.), and a PE-labeled anti-CD197 antibody (Bio legend), and NK cells: a PE-Cy7-labeled anti-CD56 antibody (BioLegend, Inc.). Antibodies against viral receptors, which were used herein, are a PE-labeled anti-CD46 antibody (eBioscience), a PE-labeled anti-CD150 antibody (Bio legend), and a PE-labeled anti-Nectin4 antibody (R&D). In addition, monocytes were defined as CD14⁺ and CD11b⁺. B cells were defined as CD19⁺ and CD3⁻. T cells were defined as CD3⁺ and CD19⁻. Neutrophils were defined as CD15⁺. NK cells were defined as CD3⁻ and CD19⁻ and CD56⁺.

### (Results)

FIG. 5 shows the GFP positive rate of cells of each blood cell lineage derived from peripheral blood. Both vectors were confirmed to exert high transduction rates in monocytes, however, MVV was confirmed to have transduction efficiency higher than SeV in B cells, T cells and neutrophils.

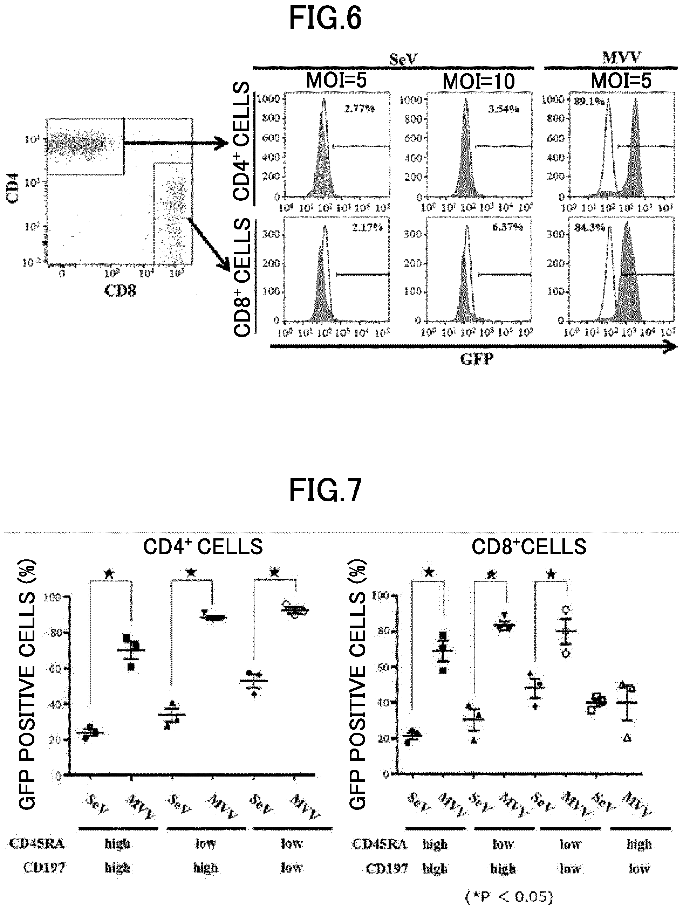

FIG. 6 shows GFP positive rates in inactivated T cells derived from cord blood. SeV was confirmed to perform no transduction even in the case of MOI=10, however, MW exerted high transduction efficiency in the case of MOI=5.

T cells were fractionated into CD45RA and CCR7 (CD197), and each fraction was examined for transduction efficiency. As shown in FIG. 7, highly efficient transduction by MW could be confirmed in fractions (CD45RA high and CD197 high) of naive T cells and stem cell-like memory T cells before exposed to antigens, and fractions (CD45RA low and CD197 high) of central memory T cells and fractions (CD45RA low and CD197 low) of effector memory T cells, which proliferate via re-exposure to antigens. On the other hand, from the results of CD8⁺ cells, no improvement in transduction efficiency by MVV was confirmed in effector T cell fractions (CD45RA high and CD197 low).

The above results indicate that MVV enables highly efficient transduction into naive T cells and stem cell-like memory T cells to which transduction has been difficult by conventional methods involving the use of Sendai virus vector. Therefore, it was strongly suggested that MVV according to this example can be an innovative therapeutic vector in a gene therapy using gene-modified T cells, and particularly a gene-modified T cell therapy. Furthermore, MW could achieve highly efficient transduction into primary cultured B cells.

### (Example 4: Establishment of iPS cells using non-transmissible gene-modified measles virus vector)

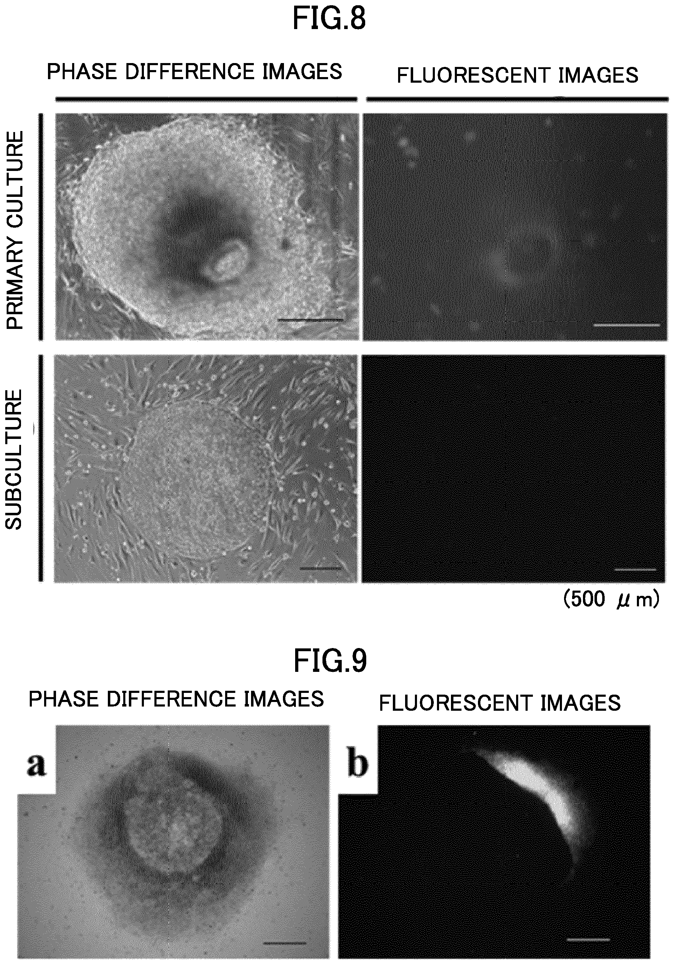

Transduction (MOI=5) was performed to BJ cells by centrifugation (room temperature, 1,200×g, 45 minutes) using MVV, the resultant was washed with PBS, and then culture solutions were exchanged. On day 7 after transduction, BJ cells were seeded on MEF cells, cultured for one day at 37°C within a 5% CO₂ incubator, and then on the next day, the medium was exchanged with a culture solution for maintaining human ES cells. Cells were then cultured at 37°C within a 5% CO₂ incubator while exchanging culture solutions every other day. On day 27 after transduction, human ES cell-like colonies were aliquoted (see FIG. 8).

Furthermore, peripheral blood-derived mononuclear cells of a healthy individual were stimulated in a KBM502 culture solution using Dynabeads (Registered Trademark) Human T-Activator CD3/CD28, and then cultured at 37°C within a 5% CO₂ incubator. On day 5 after culture, cells were recovered, transduced with reprogramming factors by centrifugation (room temperature, 1,200×g, 45 minutes) using MVV, seeded on MEF cells on the next day and then cultured at 37°C within a 5% CO₂ incubator. On day 20 after the start of culture, ES cell-like colonies (see FIG.9) were recovered, and then seeded on fresh MEF cells.

The above iPS cells established from BJ cells were subjected to an immune antibody staining method and an RT-PCR method, thereby confirming the expression of an undifferentiation marker. The immune antibody staining method was performed by immobilizing human iPS cells using 4% paraformaldehyde-phosphate buffer (NACALAI TESQUE, INC.) at 4°C for 30 minutes, treating the cells with 0.1% Triton X-100 (NACALAI TESQUE, INC.), and then blocking with 5% skim milk (NACALAI TESQUE, INC.). Staining was performed using as primary antibodies an anti-NANOG antibody (R&D), an anti-OCT3/4 antibody, an anti-SSEA-4 antibody, an anti-TRA-1-60 antibody and an anti-TRA-1-81 antibody (all antibodies produced by Santa cruz) at 4°C overnight, and a secondary antibody (anti-goat InG (Life Technologies Corporation)) at room temperature for 30 minutes. Alkaline Phospatase Detection kit (Merck Millipore) was used for alkaline phosphatase staining.

With the RT-PCR method, RNA was extracted from the above iPS cells established from BJ cells using an RNeasy mini kit (Quiagen), and then a complementary strand DNA (cDNA) was synthesized using a SuperScript III First-Strand Synthesis System for RT-PCR (Life Technologies Corporation). Subsequently, an amplification reaction was performed using Takara ExTaq polymerase (Takara Bio Inc.), and then electrophoresis was performed using 1.5% agarose gel (NACALAI TESQUE, INC.). The nucleotide sequences of primers used in the RT-PCR method are SEQ ID NO: 3 and SEQ ID NO: 4 for Endo OCT3/4, SEQ ID NO: 5 and SEQ ID NO: 6 for Endo SOX2, SEQ ID NO: 7 and SEQ ID NO: 8 for Endo KLF4, SEQ ID NO: 9 and SEQ ID NO: 10 for EndoMYC, SEQ ID NO: 11 and SEQ ID NO: 12 for NANOG, SEQ ID NO: 13 and SEQ ID NO: 14 for TERT, SEQ ID NO: 15 and SEQ ID NO: 16 for DNMT3B, SEQ ID NO: 17 and SEQ ID NO: 18 for a MV-N protein, SEQ ID NO: 19 and SEQ ID NO: 20 for MV-L protein, and SEQ ID NO: 21 and SEQ ID NO: 22 for β-actin.

Induction of triploblastic differentiation by an *in vitro* embryoid formation method was examined. The above iPS cells established from BJ cells were dissociated from MEF using an ES cell dissociation solution (mixture of Collagenase IV (Life Technologies Corporation), 0.25% trypsin (Life Technologies Corporation) and KSR), re-suspended in a culture solution for maintaining human ES cells, seeded in a non-adhesive 6-well plate, and then cultured at 37°C within a 5% CO₂ incubator. On the next day, nonadherent cells were recovered, re-suspended in a culture solution for maintaining human ES cells to which no basic FGF had been added, and then cultured at 37°C within a 5% CO₂ incubator. On day 14 after the start of culture, cells were recovered, dissociated using a 0.25% trypsin/EDTA mixture (NACALAI TESQUE, INC.), seeded on dishes coated with a 0.1% gelatin solution (NACALAI TESQUE, INC.), and then cultured for 7 days at 37°C within a 5% CO₂ incubator.

The above iPS cells established from BJ cells were confirmed to undergo triploblastic differentiation using an immune antibody staining method. Antibody used herein were an anti-alpha-fetoprotein antibody (R&D), an anti-vimentin antibody (Santa cruz) and an anti-nestin antibody (Santa cruz).

The above iPS cells established from BJ cells were subjected to a teratoma formation test. 1×10⁶ iPS cells were injected into the testis of each immunodeficient mouse (obtained from Central Institute for Experimental Animals). After 9 to 13 weeks, teratomas were extracted. Subsequently, teratoma tissues were fixed with 20% formalin (Wako Pure Chemical Industries, Ltd.), and then hematoxylin-eosin staining was performed. Moreover, the above iPS cells established from BJ cells were subjected to karyotype analysis (Chromocenter Inc.).

### (Results)

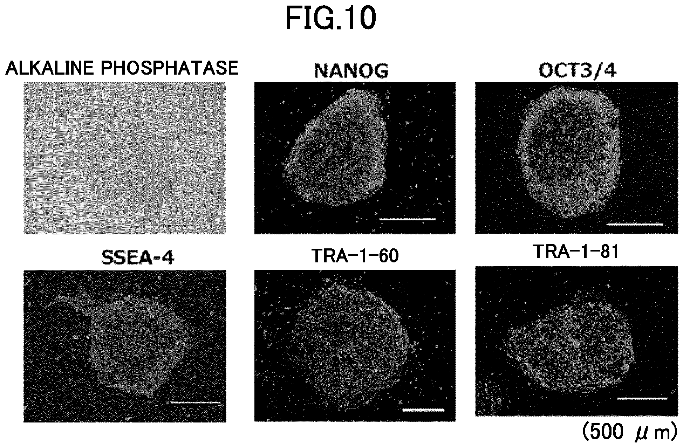

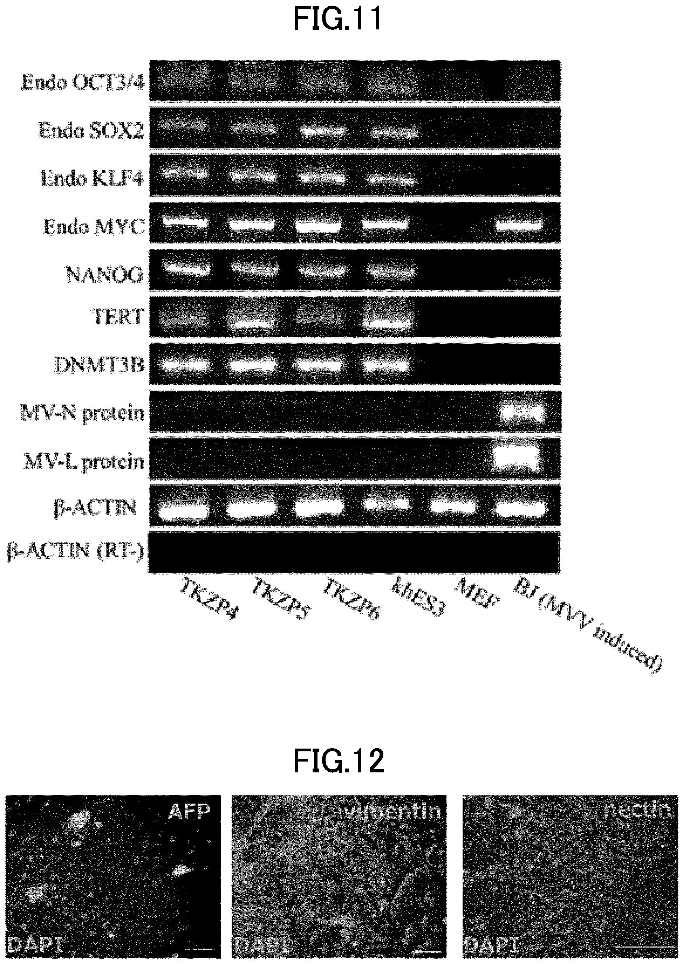

As shown in FIG. 10, the expression of alkaline phosphatase, NANOG, OCT3/4, SSEA-4, TRA-1-60 and TRA-1-81, which are expressed in undifferentiated cells, was confirmed in iPS cells. The results of the RT-PCR method are shown in FIG. 11. In each iPS cell sample, the expression of transduced OCT3/4, SOX2, KLF4 and L-MYC was confirmed. Furthermore, the expression of undifferentiation markers, NANOG, TERT and DNMT3B, was also confirmed. In addition, no expression of measles virus-derived N protein and L protein was observed. Moreover, the above iPS cells were confirmed to undergo triploblastic differentiation (see FIG. 12).

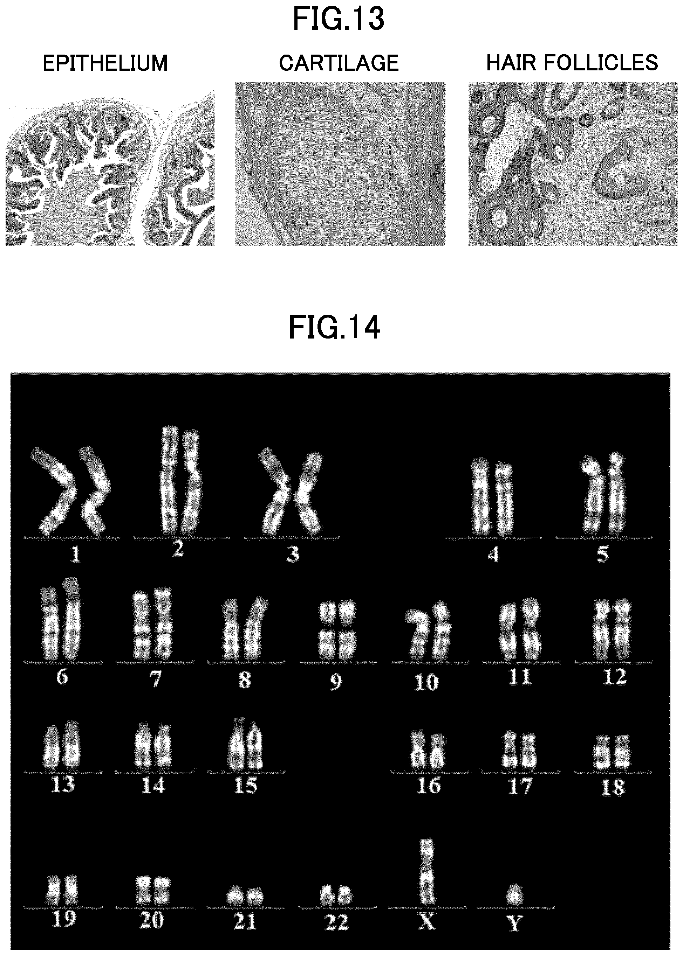

As a result of the teratoma formation test, as shown in FIG. 13, triploblastic differentiation ability was confirmed. This demonstrated the pluripotency of the above iPS cells established from BJ cells. As shown in FIG. 14, the iPS cells were found to have 46 chromosomes, and the karyotype was normal.

### (Example 5: Establishment, induction of differentiation, and analysis of iPS cells in a ground state)

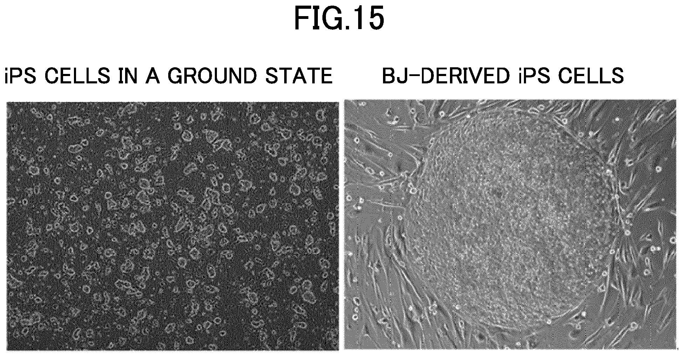

Cord blood-derived CD34 positive cells obtained from RIKEN were thawed, and then cultured using a culture solution prepared by adding SCF, TPO, and Flt3L to StemSpan at 37°C within a 5% CO₂ incubator. On the next day, transduction was performed by centrifugation (room temperature, 1,200×g, 45 minutes) using MVV, and then cells were cultured in the above culture solution for 2 days. On day 3, the cells were seeded on MEF cells, and on day 4, the culture solution was exchanged with a culture solution for maintaining human ES cells. Subsequently, culture was continued at 37°C within the 5% CO₂ incubator while exchanging culture solutions every other day. Colonies (see FIG. 15) appeared on day 14 after transduction were collected, and then dissociated into single cells with 0.25% trypsin/EDTA mixture. The cells were seeded on MEF cells and then cultured at 37°C within the 5% CO₂ incubator.

### (Results)

As shown in FIG. 15, iPS cells in a ground state could be established. iPS cells in a ground state were dissociated into single cells at 4-day intervals using a 0.25% trypsin/EDTA mixture and then subcultured. As a result, colony morphology could be maintained for even 10 or more subcultures without using Y-27632. Furthermore, through exchange of the culture solution with a culture solution for maintaining human ES cells, so as to lower the concentration of cells to be seeded on MEF cells, appearance of human ES cell-like colonies could be confirmed.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

### Industrial Applicability

The present disclosure is preferable for virus vectors and constructs for transduction to blood cells. Hence, the present disclosure is expected to be applied for immunotherapy, and particularly T cell therapy. Furthermore, the present disclosure is also preferable for preparation of pluripotent stem cells, and thus can be applied to, in addition to regenerative medicine using tissue cells obtained by induction of differentiation, disease analyses, drug discovery studies and the like using iPS cells established from cells derived from patients with intractable diseases. The present disclosure can further be applied to genome editing techniques targeting blood cells.

## Claims

1. A virus vector, comprising a genome derived from a virus belonging to the family Paramyxoviridae in which both a gene encoding an H protein and a gene encoding an F protein are modified, wherein
the genome contains a foreign gene.

2. The virus vector according to claim 1, wherein
the genome is segmented into multiple segments, and
each genome segment comprises a leader sequence and a trailer sequence.

3. The virus vector according to claim 2, wherein
the genome is segmented into two segments.

4. The virus vector according to any one of claims 1 to 3, wherein the modifications are
a deficiency in the gene encoding the H protein, or a mutation resulting from substitution, deletion, or addition of one or several amino acids of the H protein, and
a deficiency in the gene encoding the F protein, or a mutation resulting from substitution, deletion, or addition of one or several amino acids of the F protein.

5. The virus vector according to any one of claims 1 to 4, wherein
a gene encoding an M protein in the genome comprises a mutation resulting from substitution, deletion, or addition of one or several amino acids of the M protein.

6. The virus vector according to any one of claims 1 to 5, wherein
the virus belonging to the family Paramyxoviridae is measles virus.

7. The virus vector according to any one of claims 1 to 6, wherein
the foreign gene includes OCT3/4, SOX2 and KLF4.

8. A cell, wherein
the foreign gene is introduced by the virus vector according to any one of claims 1 to 7.

9. The cell according to claim 8, which is a blood cell including a hematopoietic stem cell.

10. The cell according to claim 9, wherein
the blood cell is a naive T cell, a stem cell-like memory T cell, or an undifferentiated B cell.

11. The cell according to claim 8, wherein
the foreign gene includes OCT3/4, SOX2 and KLF4, and
the cell is a pluripotent stem cell in a ground state.

12. A construct, containing
a nucleic acid that is a template for multiple genome segments derived from a virus belonging to the family Paramyxoviridae, in which a leader sequence is located at the 3' end and a trailer sequence is located at the 5' end, respectively, wherein
both the gene encoding the H protein and the gene encoding the F protein in the segmented genome are modified.


SEQUENCE LISTING

<110>  Kyushu University, National University Corporation
       Takeda, Makoto

<120>  Virus vector, cell and construct

<130>  15F079-PCT

<150>  JP 2014-225642
<151>  2014-11-05

<160>  22

<170>  PatentIn version 3.5


<210>  1
<211>  8975
<212>  DNA
<213>  Artificial sequence

<220>
<223>  Segment 1 of modified measles virus genome

<400>  1
accaaacaaa gttgggtaag gatagatcaa tcaatgatca tattctagta cacttaggat      60

tcaagatcct attatcaggg acaagagcag gattagggat atccgagggc gcgccatggc     120

gggacacctg gcttcggatt tcgccttctc gccccctcca ggtggtggag gtgatgggcc     180

aggggggccg gagccgggct gggttgatcc tcggacctgg ctaagcttcc aaggccctcc     240

tggagggcca ggaatcgggc cgggggttgg gccaggctct gaggtgtggg ggattccccc     300

atgccccccg ccgtatgagt tctgtggggg gatggcgtac tgtgggcccc aggttggagt     360

ggggctagtg ccccaaggcg gcttggagac ctctcagcct gagggcgaag caggagtcgg     420

ggtggagagc aactccgatg gggcctcccc ggagccctgc accgtcaccc ctggtgccgt     480

gaagctggag aaggagaagc tggagcaaaa cccggaggag tcccaggaca tcaaagctct     540

gcagaaagaa ctcgagcaat ttgccaagct cctgaagcag aagaggatca ccctgggata     600

tacacaggcc gatgtggggc tcaccctggg ggttctattt gggaaggtat tcagccaaac     660

gaccatctgc cgctttgagg ctctgcagct tagcttcaag aacatgtgta agctgcggcc     720

cttgctgcag aagtgggtgg aggaagctga caacaatgaa aatcttcagg agatatgcaa     780

agcagaaacc ctcgtgcagg cccgaaagag aaagcgaacc agtatcgaga accgagtgag     840

aggcaacctg gagaatttgt tcctgcagtg cccgaaaccc acactgcagc agatcagcca     900

cattgcccag cagcttgggc tcgagaagga tgtggtccga gtgtggttct gtaaccggcg     960

ccagaagggc aagcgatcaa gcagcgacta tgcacaacga gaggattttg aggctgctgg    1020

gtctcctttc tcagggggac cagtgtcctt tcctctggcc ccagggcccc attttggtac    1080

cccaggctat gggagccctc acttcactgc actgtactcc tcggtccctt tccctgaggg    1140

ggaagccttt ccccctgtct ctgtcaccac tctgggctct cccatgcatt caaactgata 1200

agacgtcgag aggccgagga ccagaacaac atccgcctac cctccatcat tgttataaaa 1260

aacttaggaa ccaggtccac acagccgcca gccaaccaac catccactcc cacgactgga 1320

gttcgaacga gatggccaca cttttgagga gcttagcatt gttcaaaaga aacaaggaca 1380

aaccacccat tacatcagga tccggtggag ccatcagagg aatcaaacac attattatag 1440

taccaattcc tggagattcc tcaattacca ctcgatccag actactggac cggttggtca 1500

ggttaattgg aaacccggat gtgagcgggc ccaaactaac aggggcacta ataggtatat 1560

tatccttatt tgtggagtct ccaggtcaat tgattcagag gatcaccgat gaccctgacg 1620

ttagcatcag gctgttagag gttgttcaga gtgaccagtc acaatctggc cttaccttcg 1680

catcaagagg taccaacatg gaggatgagg cggaccaata cttttcacat gatgatccaa 1740

gcagtagtga tcaatccagg tccggatggt tcgagaacaa ggaaatctca gatattgaag 1800

tgcaagaccc tgagggattc aacatgattc tgggtaccat tctagcccag atctgggtct 1860

tgctcgcaaa ggcggttacg gccccagaca cggcagctga ttcggagcta agaaggtgga 1920

taaagtacac ccaacaaaga agggtagttg gtgaatttag attggagaga aaatggttgg 1980

atgtggtgag gaacaggatt gccgaggacc tctctttacg ccgattcatg gtggctctaa 2040

tcctggatat caagaggaca cccgggaaca aacctaggat tgctgaaatg atatgtgaca 2100

ttgatacata tatcgtagag gcaggattag ccagttttat cctgactatt aagtttggga 2160

tagaaactat gtatcctgct cttggactgc atgaatttgc tggtgagtta tccacacttg 2220

agtccttgat gaatctttac cagcaaatgg gagaaactgc accctacatg gtaatcctag 2280

agaactcaat tcagaacaag ttcagtgcag gatcataccc tctgctctgg agctatgcca 2340

tgggagtagg agtggaactt gaaaactcca tgggaggttt gaactttggt cgatcttact 2400

ttgatccagc atattttaga ttagggcaag agatggtgag gaggtcagct ggaaaggtca 2460

gttccacatt ggcatccgaa ctcggtatca ctgccgagga tgcaaggctt gtttcagaga 2520

ttgcaatgca tactactgag gacaggatca gtagagcggt cggacccaga caagcccaag 2580

tgtcatttct acacggtgat caaagtgaga atgagctacc aggattgggg ggcaaggaag 2640

ataggagggt caaacagggt cggggagaag ccagggagag ctacagagaa accgggtcca 2700

gcagagcaag tgatgcgaga gctgcccatc ctccaaccag catgccccta gacattgaca 2760

ctgcatcgga gtcaggccaa gatccgcagg acagtcgaag gtcagctgac gccctgctca 2820

ggctgcaagc catggcagga atcttggaag aacaaggctc agacacggac acccctaggg 2880

tatacaatga cagagatctt ctagactagg tgcgagaggc cgaggaccag aacaacatcc 2940

gcctaccctc catcattgtt ataaaaaact taggaaccag gtccacacag ccgccagcca 3000

accaaccatc cactcccacg actggagccg atggcagaag agcaggcacg ccatgtcaaa 3060

aacggactgg aatgcatccg ggctctcaag gccgagccca tcggctcact ggccgtcgag 3120

gaagccatgg cagcatggtc agaaatatca gacaacccag gacaggaccg agccacctgc 3180

aaggaagagg aggcaggcag ttcgggtctc agcaaaccat gcctctcagc aattggatca 3240

actgaaggcg gtgcacctcg catccgcggt cagggatctg gagaaagcga tgacgacgct 3300

gaaactttgg gaatcccctc aagaaatctc caggcatcaa gcactgggtt acagtgttat 3360

catgtttatg atcacagcgg tgaagcggtt aagggaatcc aagatgctga ctctatcatg 3420

gttcaatcag gccttgatgg tgatagcacc ctctcaggag gagacgatga atctgaaaac 3480

agcgatgtgg atattggcga acctgatacc gagggatatg ctatcactga ccggggatct 3540

gctcccatct ctatggggtt cagggcttct gatgttgaaa ctgcagaagg aggggagatc 3600

cacgagctcc tgaaactcca atccagaggc aacaactttc cgaagcttgg gaaaactctc 3660

aatgttcctc cgcccccgaa ccccagtagg gccagcactt ccgagacacc cattaaaaag 3720

ggcacagacg cgagattggc ctcatttgga acggagatcg cgtctttatt gacaggtggt 3780

gcaacccaat gtgctcgaaa gtcaccctcg gaaccatcag ggccaggtgc acctgcgggg 3840

aatgtccccg agtgtgtgag caatgccgca ctgatacagg agtggacacc cgaatctggt 3900

accacaatct ccccgagatc ccagaataat gaagaagggg gagactatta tgatgatgag 3960

ctgttctccg atgtccaaga catcaaaaca gccttggcca aaatacacga ggataatcag 4020

aagataatct ccaagctaga atcattgctg ttattgaagg gagaagttga gtcaattaag 4080

aagcagatca acaggcaaaa tatcagcata tccaccctgg aaggacacct ctcaagcatc 4140

atgattgcca ttcctggact tgggaaggat cccaacgacc ccactgcaga tgtcgaactc 4200

aatcccgacc tgaaacccat cataggcaga gattcaggcc gagcactggc cgaagttctc 4260

aagaagcccg ttgccagccg acaactccag ggaatgacta atggacggac cagttccaga 4320

ggacagctgc tgaaggaatt tcaactaaag ccgatcggga aaaaggtgag ctcagccgtc 4380

gggtttgttc ctgacaccgg ccctgcatca cgcagtgtaa tccgctccat tataaaatcc 4440

agccggctag aggaggatcg gaagcgttac ctgatgactc tccttgatga tatcaaagga 4500

gccaacgatc ttgccaagtt ccaccagatg ctgatgaaga taataatgaa gtagctacag 4560

ctcaacttac ctgccaaccc catgccagtc gacctaatta gtacaaccta aatccattat 4620

aaaaaactta ggagcaaagt gattgcctcc taagttccac aatgacagag atctacgatt 4680

tcgacaagtc ggcatgggac atcaaagggt cgatcgctcc gatacaacct accacctaca 4740

gtgatggcag gctggtgccc caggtcagag tcatagatcc tggtctaggt gataggaagg 4800

atgaatgctt tatgtacatg tttctgctgg gggttgttga ggacagcgat cccctagggc 4860

ctccaatcgg gcgagcattc gggtccctgc ccttaggtgt tggtagatcc acagcaaaac 4920

ccgaggaact cctcaaagag gccactgagc ttgacatagt tgttagacgt acagcagggc 4980

tcaatgaaaa actggtgttc tacaacaaca ccccactaac cctcctcaca ccttggagaa 5040

aggtcctaac aacagggagt gtcttcaatg caaaccaagt gtgcaatgcg gttaatctaa 5100

taccgctgga caccccgcag aggttccgtg ttgtttatat gagcatcacc cgtctttcgg 5160

ataacgggta ttacaccgtt cccagaagaa tgctggaatt cagatcggtc aatgcagtgg 5220

ccttcaacct gctagtgacc cttaggattg acaaggcgat tggccctggg aagatcatcg 5280

acaatgcaga gcaacttcct gaggcaacat ttatggtcca catcgggaac ttcaggagaa 5340

agaagagtga agtctactct gccgattatt gcaaaatgaa aatcgaaaag atgggcctgg 5400

tttttgcact tggtgggata gggggcacca gtcttcacat tagaagcaca ggcaaaatga 5460

gcaagactct ccatgcacaa ctcgggttca agaagacctt atgttaccca ctgatggata 5520

tcaatgaaga ccttaatcgg ttactctgga ggagcagatg caagatagta agaatccagg 5580

cagttttgca gccatcagtt cctcaagaat tccgcattta cgacgacgtg atcataaatg 5640

atgaccaagg actattcaaa gttctgtaga ccgcagtgcc cagcaatacc cgaaaacgac 5700

ccccctcata atgacagcca gaaggcccgg acaaaaaagc cccctccaga agactccacg 5760

gaccaagcga gaggccagcc agcagccgac agcaagtgtg gacaccaggc ggcccaagca 5820

cagaacagcc ccgacacaag gccaccacca gccatcccaa tctgcgtcct cctcgtggga 5880

cccccgagga ccaaccccga aggtcgctcc gaacacagac caccaaccgc atccccacag 5940

ctcccgggaa aggaaccccc agcaactgga aggcccctcc ccccctcccc caacgcaaga 6000

accccacaac cgaaccgcac aagcgaccga ggtgacccaa ccgcaggcat ccgactcctt 6060

agacagatcc tctccccccg gcatactaaa caaaacttag ggccaaggaa cacacacact 6120

cgacagaacc cagaccccgg cccgcggcac cgcgccccca ccccccgaaa accagaggga 6180

gcccccaacc aaacccgccg gcccccccgg tgcccacagg taggcacacc aacccccgac 6240

cagacccagc acccagccac cgacaatcca agacgggggg ccccccccaa aaaaaggccc 6300

ccaggggccg acagccagca tcgatgtaca acatgatgga gacggagctg aagccgccgg 6360

gcccgcagca aacttcgggg ggcggcggcg gcaactccac cgcggcggcg gccggcggca 6420

accagaaaaa cagcccggac cgcgtcaagc ggcccatgaa tgccttcatg gtgtggtccc 6480

gcgggcagcg gcgcaagatg gcccaggaga accccaagat gcacaactcg gagatcagca 6540

agcgcctggg cgccgagtgg aaacttttgt cggagacgga gaagcggccg ttcatcgacg 6600

aggctaagcg gctgcgagcg ctgcacatga aggagcaccc ggattataaa taccggcccc 6660

ggcggaaaac caagacgctc atgaagaagg ataagtacac gctgcccggc gggctgctgg 6720

cccccggcgg caatagcatg gcgagcgggg tcggggtggg cgccggcctg ggcgcgggcg 6780

tgaaccagcg catggacagt tacgcgcaca tgaacggctg gagcaacggc agctacagca 6840

tgatgcagga ccagctgggc tacccgcagc acccgggcct caatgcgcac ggcgcagcgc 6900

agatgcagcc catgcaccgc tacgacgtga gcgccctgca gtacaactcc atgaccagct 6960

cgcagaccta catgaacggc tcgcccacct acagcatgtc ctactcgcag cagggcaccc 7020

ctggcatggc tcttggctcc atgggttcgg tggtcaagtc cgaggccagc tccagccccc 7080

ctgtggttac ctcttcctcc cactccaggg cgccctgcca ggccggggac ctccgggaca 7140

tgatcagcat gtatctcccc ggcgccgagg tgccggaacc cgccgccccc agcagacttc 7200

acatgtccca gcactaccag agcggcccgg tgcccggcac ggccattaac ggcacactgc 7260

ccctctcaca catgtgataa cggtcctccc atatcattgg agaggttgga cgtagggaca 7320

aatctgggga atgcaattgc taagttggag gatgccaagg aattgttgga gtcatcggac 7380

cagatattga ggagtatgaa aggtttatcg agcactagca tagtttacat cctgattgca 7440

gtgtgtcttg gagggttgat agggatcccc gctttaatat gttgctgcag ggggcgctgt 7500

aacaaaaagg gagaacaagt tggtatgtca agaccaggcc taaagcctga tcttacaggg 7560

acatcaaaat cctatgtaag gtcgctctga tcctctacaa ctcttgaaac acagatttcc 7620

cacaagtctc ctcttcgtca tcaagcaacc accgcatcca gcatcaagcc cacctgaaat 7680

tgtctccggc ttccctctgg ccgaacgata tcggtagtta attaaaactt agggtgcaag 7740

atcatccaca atggactacg actcgtacca gcactatttc tacgactatg actgcgggga 7800

ggatttctac cgctccacgg cgcccagcga ggacatctgg aagaaattcg agctggtgcc 7860

atcgcccccc acgtcgccgc cctggggctt gggtcccggc gcaggggacc cggcccccgg 7920

gattggtccc ccggagccgt ggcccggagg gtgcaccgga gacgaagcgg aatcccgggg 7980

ccactcgaaa ggctggggca ggaactacgc ctccatcata cgccgtgact gcatgtggag 8040

cggcttctcg gcccgggaac ggctggagag agctgtgagc gaccggctcg ctcctggcgc 8100

gccccggggg aacccgccca aggcgtccgc cgccccggac tgcactccca gcctcgaagc 8160

cggcaacccg gcgcccgccg ccccctgtcg ctgggcgaac ccaagaccca ggcctgctcc 8220

gggtccgaga gcccaagcga ctcggagaat gaagaaattg atgttgtgac agtagagaag 8280

aggcagtctc tgggtattcg gaagccggtc accatcacgg tgcgagcaga ccccctggat 8340

ccctgcatga agcatttcca catctccatc catcagcaac agcacaacta tgctgcccgt 8400

tttcctccag aaagctgctc ccaagaagag gcttcagaga ggggtcccca agaagaggtt 8460

ctggagagag atgctgcagg ggaaaaggaa gatgaggagg atgaagagat tgtgagtccc 8520

ccacctgtag aaagtgaggc tgcccagtcc tgccacccca aacctgtcag ttctgatact 8580

gaggatgtga ccaagaggaa gaatcacaac ttcctggagc gcaagaggcg gaatgacctg 8640

cgttcgcgat tcttggcgct gagggaccag gtgcccaccc tggccagctg ctccaaggcc 8700

cccaaagtag tgatcctaag caaggccttg gaatacttgc aagccctggt gggggctgag 8760

aagaggatgg ctacagagaa aagacagctc cgatgccggc agcagcagtt gcagaaaaga 8820

attgcatacc tcactggcta ctaatagcgc tctgattaag gattaattgg ttgaactccg 8880

gaaccctaat cctgccctag gtagttaggc attatttgca atatattaaa gaaaactttg 8940

aaaatacgaa gtttctattc ccagctttgt ctggt 8975

<210> 2
<211> 11658
<212> DNA
<213> Artificial sequence

<220>
<223> Segment 2 of modified measles virus genome

<400> 2
accaaacaaa gttgggtaag gatagatcaa tcaatgatca tattctagta cacttaggat 60

tcaagatcct attatcaggg acaagagcag gattagggat atccgagggc gcgccatggt 120

gagcaagggc gaggagctgt tcaccggggt ggtgcccatc ctggtcgagc tggacggcga 180

cgtaaacggc cacaagttca gcgtgtccgg cgagggcgag ggcgatgcca cctacggcaa 240

gctgaccctg aagttcatct gcaccaccgg caagctgccc gtgccctggc ccaccctcgt 300

gaccaccctg acctacggcg tgcagtgctt cagccgctac cccgaccaca tgaagcagca 360

cgacttcttc aagtccgcca tgcccgaagg ctacgtccag gagcgcacca tcttcttcaa 420

ggacgacggc aactacaaga cccgcgccga ggtgaagttc gagggcgaca ccctggtgaa 480

ccgcatcgag ctgaagggca tcgacttcaa ggaggacggc aacatcctgg ggcacaagct 540

ggagtacaac tacaacagcc acaacgtcta tatcatggcc gacaagcaga agaacggcat 600

caaggtgaac ttcaagatcc gccacaacat cgaggacggc agcgtgcagc tcgccgacca 660

ctaccagcag aacaccccca tcggcgacgg ccccgtgctg ctgcccgaca accactacct 720

gagcacccag tccgccctga gcaaagaccc caacgagaag cgcgatcaca tggtcctgct 780

ggagttcgtg accgccgccg ggatcactct cggcatggac gagctgtaca agtaagacgt 840

cgagaggccg aggaccagaa caacatccgc ctaccctcca tcattgttat aaaaaactta 900

ggaaccaggt ccacacagcc gccagccaac caaccatcca ctcccacgac tggagttcga 960

agcagatggc tgtcagcgac gcgctgctcc catctttctc cacgttcgcg tctggcccgg 1020

cgggaaggga gaagacactg cgtcaagcag gtgccccgaa taaccgctgg cgggaggagc 1080

tctcccacat gaagcgactt cccccagtgc ttcccggccg cccctatgac ctggcggcgg 1140

cgaccgtggc cacagacctg gagagcggcg gagccggtgc ggcttgcggc ggtagcaacc 1200

tggcgcccct acctcggaga gagaccgagg agttcaacga tctcctggac ctggacttta 1260

ttctctccaa ttcgctgacc catcctccgg agtcagtggc cgccaccgtg tcctcgtcag 1320
cgtcagcctc ctcttcgtcg tcgccgtcga gcagcggccc tgccagcgcg ccctccacct 1380
gcagcttcac ctatccgatc cgggccggga acgacccggg cgtggcgccg ggcggcacgg 1440
gcggaggcct cctctatggc agggagtccg ctccccctcc gacggctccc ttcaacctgg 1500
cggacatcaa cgacgtgagc cccctcggcg gcttcgtggc cgagctcctg cggccagaat 1560
tggacccggt gtacattccg ccgcagcagc cgcagccgcc aggtggcggg ctgatgggca 1620
agttcgtgct gaaggcgtcg ctgagcgccc ctggcagcga gtacggcagc ccgtcggtca 1680
tcagcgtcag caaaggcagc cctgacggca gccacccggt ggtggtggcg ccctacaacg 1740
gcgggccgcc gcgcacgtgc cccaagatca agcaggaggc ggtctcttcg tgcacccact 1800
tgggcgctgg accccctctc agcaatggcc accggccggc tgcacacgac ttccccctgg 1860
ggcggcagct ccccagcagg actaccccga ccctgggtct tgaggaagtg ctgagcagca 1920
gggactgtca ccctgccctg ccgcttcctc ccggcttcca tccccacccg gggcccaatt 1980
acccatcctt cctgcccgat cagatgcagc cgcaagtccc gccgctccat taccaagagc 2040
tcatgccacc cggttcctgc atgccagagg agcccaagcc aaagagggga agacgatcgt 2100
ggccccggaa aaggaccgcc acccacactt gtgattacgc gggctgcggc aaaacctaca 2160
caaagagttc ccatctcaag gcacacctgc gaacccacac aggtgagaaa ccttaccact 2220
gtgactggga cggctgtgga tggaaattcg cccgctcaga tgaactgacc aggcactacc 2280
gtaaacacac ggggcaccgc ccgttccagt gccaaaaatg cgaccgagca ttttccaggt 2340
cggaccacct cgccttacac atgaagaggc atttttaata ggacgtcgag aggccgagga 2400
ccagaacaac atccgcctac gatatcggta gttaattaaa acttagggtg caagatcatc 2460
cacaatgtca ccacaacgag accgaataaa tgccttctac aaagacaacc cacatcctaa 2520
gggaagtagg atagttatta acagagaaca tcttatgatt gatagacctt atgttttgct 2580
ggctgttcta ttcgtcatgt ttctgagctt gatcgggttg ctagccattg caggcattag 2640
actccatcgt gcagccatct acaccgcaga gatccataag agcctcagca ccaatctaga 2700
tgtaactaac tcgatcgagc atcaggtcaa ggacgtgctg acaccactct tcaagatcat 2760
tggtgatgaa gtgggcctga ggacacctca gagattcact gacctagtga aattcatctc 2820
tgacaaaatt aaattcctta atccggatag ggagtacgac ttcagagatc tcacttggtg 2880
tatcaacccg ccagagagaa tcaaattgga ttatgatcaa tactgtgcag atgtggctgc 2940
tgaagaactc atgaatgcat tggtgaactc aactctactg gaggccaggg caaccaatca 3000
gttcctagct gtctcaaagg gaaactgctc agggcccact acaatcagag gtcaattctc 3060
aaacatgtcg ctgtccctgt tggacttgta tttaagtcga ggttacaatg tgtcatctat 3120
agtcactatg acatcccagg gaatgtacgg gggaacttac ctagtgggaa agcctaatct 3180

gagcagtaaa gggtcagagt tgtcacaact gagcatgcac cgagtgtttg aagtaggggt 3240

tatcagaaat ccgggtttgg gggctccggt gttccatatg acaaactatt ttgagcaacc 3300

agtcagtaat gatttcagca actgcatggt ggctttgggg gagcttaaat tcgcagccct 3360

ctgtcacagg gaagattcta tcacaattcc ctatcagggg tcagggaaag gtgtcagctt 3420

ccagctcgtc aagctaggtg tctggaaatc cccaaccgac atgcgatcct gggtccccct 3480

atcaacggat gatccagtga tagataggct ttatctctca tctcacagag gtgttatcgc 3540

tgacaatcaa gcaaaatggg ctgtcccgac aacacggaca gatgacaagt tgcgaatgga 3600

gacatgcttc cagcaggcgt gtaagggtaa aatacaagca ctctgcgaga atcccgagtg 3660

ggcaccattg aaggataaca ggattccttc atacggggtc ttgtctgttg acctgagtct 3720

gacagttgag cttaaaatca aaattgcttc aggattcggg ccattgatca cacacggttc 3780

agggatggac ctatacaaaa ccaaccacaa caatgtgtat tggctgacta tcccgccaat 3840

gaagaaccta gccttaggtg taatcaacac attggagtgg ataccgagat tcaaggttag 3900

tccctatctc ttcactgttc caatcaagga agcaggcggc gactgccatg ccccaacata 3960

cctacctgcg gaggtggatg gtgatgtcaa actcagttcc aatctggtaa ttctacctgg 4020

tcaggatctc caatatgttt tggcaaccta cgatacttcc agggttgaac atgctgtggt 4080

ttattatgtt tacagcccaa gccgctcatt ttcttacttt tatcctttta ggttgcctat 4140

aaagggggtc ccaatcgaat tacaagtgga atgcttcaca tgggacaaaa aactctggtg 4200

ccgtcacttc tgtgtgcttg cggactcaga atctggtgga catatcactc actctgggat 4260

ggtgggcatg ggagtcagct gcacagtcac tcgggaagat ggaaccaatc gcagataggg 4320

ctgccagtga accgatcaca tgatgtcacc cagacatcag gcatacccac tagtgtgaaa 4380

tagacatcag aattaagaaa aacgtagggt ccaagtggtt tcccgttggc cggccatggc 4440

ggacgaggag aagctgccgc ccggctggga gaagcgcatg agccgcagct caggccgagt 4500

gtactacttc aaccacatca ctaacgccag ccagtgggag cggcccagcg gcaacagcag 4560

cagtggtggc aaaaacgggc agggggagcc tgccagggtc cgctgctcgc acctgctggt 4620

gaagcacagc cagtcacggc ggccctcgtc ctggcggcag gagaagatca cccggaccaa 4680

ggaggaggcc ctggagctga tcaacggcta catccagaag atcaagtcgg gagaggagga 4740

ctttgagtct ctggcctcac agttcagcga ctgcagctca gccaaggcca ggggagacct 4800

gggtgccttc agcagaggtc agatgcagaa gccatttgaa gacgcctcgt ttgcgctgcg 4860

gacgggggag atgagcgggc ccgtgttcac ggattccggc atccacatca tcctccgcac 4920

tgagtgacgg accgacccac tagtgtgaaa tagacatcag aattaagaaa aacgtagggt 4980

ccaagtggtt tcccgttatg gactcgctat ctgtcaacca gatcttatac cctgaagttc 5040

acctagatag cccgatagtt accaataaga tagtagctat cctggagtat gctcgagtcc 5100

ctcacgctta cagcctggag gaccctacac tgtgtcagaa catcaagcac cgcctaaaaa 5160

acggattctc caaccaaatg attataaaca atgtggaagt tgggaatgtc atcaagtcca 5220

agcttaggag ttatccggcc cactctcata ttccatatcc aaattgtaat caggatttat 5280

ttaacataga agacaaagag tcaacaagga agatccgtga gctcctaaaa aagggaaatt 5340

cgctgtactc caaagtcagt gataaggttt tccaatgcct gagggacact aactcacggc 5400

ttggcctagg ctccgaattg agggaggaca tcaaggagaa aattattaac ttgggagttt 5460

acatgcacag ctcccaatgg tttgagccct ttctgttttg gtttacagtc aagactgaga 5520

tgaggtcagt gattaaatca caaacccata cttgccatag gaggagacac acacctgtat 5580

tcttcactgg tagttcagtt gagctgttaa tctctcgtga ccttgttgct ataatcagta 5640

aggaatctca acatgtatat tacctgacgt ttgaactggt tttgatgtat tgtgatgtca 5700

tagaggggag gttaatgaca gagaccgcta tgaccattga tgctaggtat gcagaacttc 5760

taggaagagt cagatacatg tggaaactga tagatggttt cttccctgca ctcgggaatc 5820

caacttatca aattgtagcc atgctggagc cactttcact tgcttacctg caactgaggg 5880

atataacagt agaactcaga ggtgctttcc ttaaccactg ctttactgaa atacatgatg 5940

ttcttgacca aaacgggttt tctgatgaag gtacttatca tgagttaatt gaagccctag 6000

attacatttt cataactgat gacatacatc tgacagggga gattttctca tttttcagaa 6060

gtttcggcca ccccagactt gaagcagtaa cggctgctga aaatgtcagg aaatacatga 6120

atcagcctaa agtcattgtg tatgagactc tgatgaaagg tcatgccata ttttgtggaa 6180

tcataatcaa cggctatcgt gacaggcacg gaggcagttg gccacccctg accctccccc 6240

tgcatgctgc agacacaatc cggaatgctc aagcttcagg tgaagggtta acacatgagc 6300

agtgcgttga taactggaaa tcatttgctg gagtgagatt tggctgtttt atgcctctta 6360

gcctggacag tgatctgaca atgtacctaa aggacaaggc acttgctgct ctccaaaggg 6420

aatgggattc agtttacccg aaagagttcc tgcgttacga tcctcccaag ggaaccgggt 6480

cacggaggct tgtagatgtt ttccttaatg attcgagctt tgacccatat gatatgataa 6540

tgtatgtcgt aagtggagcc tacctccatg accctgagtt caacctgtct tacagcctga 6600

aagaaaagga gatcaaggaa acaggtagac ttttcgctaa aatgacttac aaaatgaggg 6660

catgccaagt gatcgctgaa aatctaatct caaacgggat tggcaagtat tttaaggaca 6720

atgggatggc caaggatgag cacgatttga ctaaggcact ccacactctg gctgtctcag 6780

gagtccccaa agatctcaaa gaaagtcaca ggggggggcc agtcttaaaa acctactccc 6840

gaagcccagt ccacacaagt accaggaacg ttaaagcaga aaaagggttt gtaggattcc 6900

ctcatgtaat tcggcagaat caagacactg atcatccgga gaatatagaa acctacgaga 6960

cagtcagcgc atttatcacg actgatctca agaagtactg ccttaattgg agatatgaga 7020

ccatcagctt atttgcacag aggctaaatg agatttacgg attaccctca ttttttcagt 7080

ggctgcataa gaggcttgaa acctctgtcc tctatgtaag tgaccctcat tgcccccccg 7140

accttgacgc ccatgtcccg ttatgcaaag tccccaatga ccaaatcttc atcaagtacc 7200

ctatgggagg tatagaaggg tattgtcaga agctgtggac catcagcacc attccctact 7260

tatacctggc tgcttatgag agccgggtaa ggattgcttc gttagtgcaa ggggacaatc 7320

agaccatagc cgtaacaaaa agggtaccca gcacatggca ttacaacctt aagaaacggg 7380

aagctgctag agtaactaga gattactttg taattcttag gcaaaggcta catgacattg 7440

gccatcacct caaggcaaat gagacaattg tttcatcaca tttttttgtc tattcaaaag 7500

gaatatatta tgatgggcta cttgtgtccc aatcactcaa gagcatcgca agatgtgtat 7560

tctggtcaga gactatagtt gatgaaacaa gggcggcatg cagtaatatt gctacaacaa 7620

tggccaaaag cattgagaga ggttatgacc gttatcttgc atattccctg aacgtcctaa 7680

aagtgataca gcaaattttg atctcccttg gcttcacaat caattcaacc atgacccggg 7740

atgtagtcat acccctcctc acaaacaacg atctcttaat aaggatggca ctgttgcccg 7800

ctcctattgg gggtatgaat tatctgaata tgagcaggct gtttgtcaga aacatcggtg 7860

atccagtaac atcatcaatt gctgatctca agagaatgat tctcgcatca ctaatgcctg 7920

aagagaccct ccatcaagta atgacacaac aaccggggga ctcttcattc ctagactggg 7980

ctagcgaccc ttactcagca aatcttgtat gcgtccagag catcactaga ctcctcaaga 8040

acataactgc aaggtttgtc ctgatccaca gtccaaaccc aatgttaaaa gggttattcc 8100

atgatgacag caaagaagag gacgagagac tggcggcatt cctcatggac aggcatatta 8160

tagtacctag ggcagctcat gaaatcctgg atcatagtgt cacaggggca agagagtcta 8220

ttgcaggcat gctagatacc acaaaaggcc tgattcgagc cagcatgagg aagggggggt 8280

taacctctcg agtgataacc agattgtcca attatgacta tgaacaattt agagcaggga 8340

tggtgctatt gacaggaaga aagagaaatg tcctcattga caaagagtca tgttcagtgc 8400

agctggctag agccctaaga agccatatgt gggcaagact agctcgagga cggcctattt 8460

acggccttga ggtccctgat gtactagaat ctatgcgagg ccaccttatt cggcgtcatg 8520

agacatgtgt catctgcgag tgtggatcag tcaactacgg atggtttttt gtcccctcgg 8580

gttgccaact ggatgatatt gacaaggaaa catcatcctt gagagtccca tatattggtt 8640

ctaccactga tgagagaaca gacatgaagc tcgccttcgt aagagcccca agtagatcct 8700

tgcgatctgc cgttagaata gcaacagtgt actcatgggc ttacggtgat gatgatagct 8760

cttggaacga agcctggttg ttggcaaggc aaagggccaa tgtgagcctg gaggagctaa 8820

gggtgatcac tcccatctcg acttcgacta atttagcgca taggttgagg gatcgtagca 8880

ctcaagtgaa atactcaggt acatcccttg tccgagtggc aaggtatacc acaatctcca 8940

acgacaatct ctcatttgtc atatcagata agaaggttga tactaacttt atataccaac 9000

aaggaatgct tctagggttg ggtgttttag aaacattgtt tcgactcgag aaagatactg 9060

gatcatctaa cacggtatta catcttcacg tcgaaacaga ttgttgcgtg atcccgatga 9120

tagatcatcc caggataccc agctcccgca agctagagct gagggcagag ctatgtacca 9180

acccattgat atatgataat gcacctttaa ttgacagaga tgcaacaagg ctatacaccc 9240

agagccatag gaggcacctt gtggaatttg ttacatggtc cacaccccaa ctatatcaca 9300

ttctagctaa gtccacagca ctatctatga ttgacctggt aacaaaattt gagaaggacc 9360

atatgaatga aatttcagct ctcatagggg atgacgatat caatagtttc ataactgagt 9420

ttctgcttat agagccaaga ttattcacca tctacttggg ccagtgtgca gccatcaatt 9480

gggcatttga tgtacattat catagaccat cagggaaata tcagatgggt gagctgttgt 9540

cttcgttcct ttctagaatg agcaaaggag tgtttaaggt gcttgtcaat gctctaagcc 9600

acccaaagat ctacaagaaa ttctggcatt gtggtattat agagcctatc catggtcctt 9660

cacttgatgc tcaaaacttg cacacaactg tgtgcaacat ggtttacaca tgctatatga 9720

cctacctcga cctgttgttg aatgaagagt tagaagagtt cacatttctt ttgtgtgaaa 9780

gcgatgagga tgtagtaccg gacagattcg acaacatcca ggcaaaacac ttgtgtgttc 9840

tggcagattt gtactgtcaa ccagggacct gcccaccgat tcgaggtcta aggccggtag 9900

agaaatgtgc agttctaacc gatcatatca aggcagaggc taggttatct ccagcaggat 9960

cttcgtggaa cataaatcca attattgtag accattactc atgctctctg acttatctcc 10020

gtcgaggatc tatcaaacag ataagattga gagttgatcc aggattcatt tttgacgccc 10080

tcgctgaggt aaatgtcagt cagccaaagg tcggcagcaa caacatctca aatatgagca 10140

tcaaggattt cagacctcca cacgatgatg ttgcaaaatt gctcaaagat atcaacacaa 10200

gcaagcacaa tcttcccatt tcagggggta gtctcgccaa ttatgaaatc catgctttcc 10260

gcagaatcgg gttaaactca tctgcttgct acaaagctgt tgagatatca acattaatta 10320

ggagatgcct tgagccaggg gaagacggct tgttcttggg tgaggggtcg ggttctatgt 10380

tgatcactta taaggagata ctaaaactaa acaagtgctt ctataatagt ggggtttccg 10440

ccaattctag atctggtcaa agggaattag caccctatcc ctccgaagtt ggccttgtcg 10500

aacacagaat gggagtaggt aatattgtca aggtgctctt taacgggagg cccgaagtca 10560

cgtgggtagg cagtatagat tgcttcaatt tcatagtcag taatatccct acctctagtg 10620

tggggtttat ccattcagat atagagacct tacctaacaa agatactata gagaagctag 10680

aggaattggc agccatctta tcgatggctc tactccttgg caaaatagga tcaatactgg 10740

tgattaagct tatgcctttc agcggggatt ttgttcaggg atttataagc tatgtagggt 10800

ctcattatag agaagtgaac cttgtctacc ctaggtacag caacttcata tctactgaat 10860

cttatttagt tatgacagat ctcaaagcta accggctaat gaatcctgaa aagatcaagc 10920

agcagataat tgaatcatct gtacggactt cacctggact tataggtcac atcctatcca 10980

ttaagcaact aagctgcata caagcaattg tgggaggcgc ggttagtaga ggtgatatca 11040

accctattct gaaaaaactt acacctatag agcaggtgct gatcagttgc gggttggcaa 11100

ttaacggacc taaactgtgc aaagaattaa tccaccatga tgttgcctca gggcaagatg 11160

gattgcttaa ctctatactc atcctctaca gggagttggc aagattcaaa gacaaccaaa 11220

gaagtcaaca agggatgttc cacgcttacc ccgtattggt aagtagtagg caacgagaac 11280

ttgtatctag gatcactcgc aaatttgggg ggcatattct tctttactcc gggaacagaa 11340

agttgataaa tcggtttatc cagaatctca agtccggtta tctagtacta gacttacacc 11400

agaatatctt cgttaagaat ctatccaagt cagagaaaca gattattatg acggggggtt 11460

taaaacgtga gtgggttttt aaggtaacag tcaaggagac caaagaatgg tacaagttag 11520

tcggatacag cgctctgatt aaggattaat tggttgaact ccggaaccct aatcctgccc 11580

taggtagtta ggcattattt gcaatatatt aaagaaaact ttgaaaatac gaagtttcta 11640

ttcccagctt tgtctggt 11658

<210> 3
<211> 24
<212> DNA
<213> Artificial sequence

<220>
<223> PCR primer for Endo OCT3/4, S1165

<400> 3
gacaggggga ggggaggagc tagg 24

<210> 4
<211> 26
<212> DNA
<213> Artificial sequence

<220>
<223> PCR primer for Endo OCT3/4, AS1283

<400> 4
cttccctcca accagttgcc ccaaac 26

<210> 5
<211> 26
<212> DNA
<213> Artificial sequence

<220>
<223>  PCR primer for Endo SOX2, S1430

<400>  5
gggaaatggg aggggtgcaa aagagg                                          26


<210>  6
<211>  26
<212>  DNA
<213>  Artificial sequence

<220>
<223>  PCR primer for Endo SOX2, AS1555

<400>  6
ttgcgtgagt gtggatggga ttggtg                                          26


<210>  7
<211>  25
<212>  DNA
<213>  Artificial sequence

<220>
<223>  PCR primer for Endo KLF4, S1128

<400>  7
acgatcgtgg ccccggaaaa ggacc                                           25


<210>  8
<211>  28
<212>  DNA
<213>  Artificial sequence

<220>
<223>  PCR primer for Endo KLF4, AS1826

<400>  8
tgattgtagt gctttctggc tgggctcc                                        28


<210>  9
<211>  26
<212>  DNA
<213>  Artificial sequence

<220>
<223>  PCR primer for Endo MYC, S253

<400>  9
gcgtcctggg aagggagatc cggagc                                          26


<210>  10
<211>  26
<212>  DNA
<213>  Artificial sequence

<220>
<223>  PCR primer for Endo MYC, AS555

<400>  10
ttgaggggca tcgtcgcggg aggctg                                          26


<210>  11
<211>  25
<212>  DNA
<213>  Artificial sequence

<220>
<223>  PCR primer for NANOG, 968S

<400>  11
cagccccgat tcttccacca gtccc                                           25


<210>  12
<211>  25
<212>  DNA
<213>  Artificial sequence

<220>
<223>  PCR primer for NANOG, 1334AS

<400>  12
cggaagattc ccagtcgggt tcacc                                           25


<210>  13
<211>  26
<212>  DNA
<213>  Artificial sequence

<220>
<223>  PCR primer for TERT, S3234

<400>  13
cctgctcaag ctgactcgac accgtg                                          26


<210>  14
<211>  25
<212>  DNA
<213>  Artificial sequence

<220>
<223>  PCR primer for TERT, AS3713

<400>  14
ggaaaagctg gccctggggt ggagc                                           25


<210>  15
<211>  25
<212>  DNA
<213>  Artificial sequence

<220>
<223>  PCR primer for DNMT3B, S2502

<400>  15
tgctgctcac agggcccgat acttc                                           25

<210>  16
<211>  28
<212>  DNA
<213>  Artificial sequence

<220>
<223>  PCR primer for DNMT3B, S2716

<400>  16
tcctttcgag ctcagtgcac cacaaaac                                          28


<210>  17
<211>  18
<212>  DNA
<213>  Artificial sequence

<220>
<223>  PCR primer for MV-N protein, FW

<400>  17
gaactcggta tcactgcc                                                     18


<210>  18
<211>  18
<212>  DNA
<213>  Artificial sequence

<220>
<223>  PCR primer for MV-N protein, RV

<400>  18
tcctggtagc tcattctc                                                     18


<210>  19
<211>  20
<212>  DNA
<213>  Artificial sequence

<220>
<223>  PCR primer for MV-L protein, FW

<400>  19
cacggtatta catcttcacg                                                   20


<210>  20
<211>  20
<212>  DNA
<213>  Artificial sequence

<220>
<223>  PCR primer for MV-L protein, RV

<400>  20
gcatctctgt caattaaagg                                                   20


<210>  21

<211>  21
<212>  DNA
<213>  Artificial sequence

<220>
<223>  PCR primer for beta-actin, S756

<400>  21
gcaagagatg gccacggctg c                                                21


<210>  22
<211>  22
<212>  DNA
<213>  Artificial sequence

<220>
<223>  PCR primer for beta-actin, AS1033

<400>  22
tctccttctg catcctgtcg gc                                               22